# EUROPÄISCHE PATENTSCHRIFT

(11) **EP 3 653 361 B1**
(45) Veröffentlichungstag und Bekanntmachung des Hinweises auf die Patenterteilung: **14.08.2024**
(21) Anmeldenummer: 19181265.0
(22) Anmeldetag: 19.06.2019
(51) Int. Cl.: B29C 64/112, B29C 64/124, C08J 3/24, B29C 48/00, B29C 45/00, B29C 43/00, B33Y 10/00, B33Y 30/00, B33Y 70/00, A61L 27/12, A61L 27/20, A61L 27/22, A61L 27/38, A61L 27/50

(54) **3D-DRUCKER, EXTRUDER, SPRITZGIESSER, FORMPRESSE, VERFAHREN ZUM 3D-DRUCK EINES BIOLOGISCHEN MATERIALS UND ÜBER DAS VERFAHREN HERGESTELLTES BIOLOGISCHES MATERIAL**
3D PRINTER, EXTRUDER, INJECTION MOULDING MACHINE, COMPRESSION MOULDING MACHINE, METHOD FOR 3D PRINTING OF A BIOLOGICAL MATERIAL AND BIOLOGICAL MATERIAL PRODUCED USING THE METHOD
IMPRIMANTE 3D, EXTRUDEUSE, MACHINE THE MOULAGE PAR INJECTION, PRESSE À MOULER, PROCÉDÉ D'IMPRESSION 3D D'UN MATÉRIAU BIOLOGIQUE ET MATÉRIAU BIOLOGIQUE FABRIQUÉ SELON LEDIT PROCÉDÉ

(30) Priorität: 20.06.2018 DE 102018210048
(43) Veröffentlichungstag der Anmeldung: 20.05.2020
(73) Patentinhaber: Fraunhofer-Gesellschaft zur Förderung der angewandten Forschung e.V., 80686 München (DE)
(72) Erfinder: SCHWARZ, Dr. Oliver, 70569 Stuttgart (DE); WENDLANDT, Tim, 70569 Stuttgart (DE)
(74) Vertreter: Pfenning, Meinig & Partner mbB

(56) Entgegenhaltungen:
- US-A1- 2016 279 868
- AJAY TIJORE ET AL: "Contact guidance for cardiac tissue engineering using 3D bioprinted gelatin patterned hydrogel", BIOFABRICATION, vol. 10, no. 2, 025003, 12 January 2018 (2018-01-12), UK, pages 1 - 14, XP055683321, ISSN: 1758-5082, DOI: 10.1088/1758-5090/aaa15d

## Beschreibung

Es wird ein 3D-Drucker bereitgestellt, der eine Kammer, eine Dosiereinheit zum Inkontaktbringen von mindestens einem Enzym mit einem in der Kammer befindlichen Material und eine Steuerungseinheit enthält. Der 3D-Drucker zeichnet sich dadurch aus, dass die Dosiereinheit mindestens ein Enzym enthält, das sich zur Katalyse einer kovalenten (chemischen) Verbindung von biologischem Material eignet und die Steuerungseinheit dazu konfiguriert ist, das Inkontaktbringen des mindestens einen Enzyms mit einem in der Kammer befindlichen biologischen Material zu veranlassen. Über den erfindungsgemäßen 3D-Drucker können auf einfache und schnelle Art und Weise dreidimensionale biologische Materialien gedruckt werden, die gegenüber vergleichbaren biologischen Materialien verbesserte (biologische, chemische und mechanische) Eigenschaften aufweisen. Ein entsprechendes Verfahren zum 3D-Druck eines biologischen Materials wird vorgestellt und ein über das Verfahren herstellbares bzw. hergestelltes biologisches Material bereitgestellt.

Die bahnbrechende Technologie der 3D-Drucktechnik wird die Produktionsprozesse des 21. Jahrhunderts massiv verändern. Es sind mehrere Verfahren bekannt, mit denen ein 3D-Druck durchgeführt werden kann. Ein bekanntes Verfahren ist das sog. Selektive Lasersintern (SLS). Unter "Sintern" wird hierbei ein sog. "rapid prototyping"-Verfahren verstanden, bei dem die Herstellung von 3D-Modellen mithilfe eines Laserstrahls erfolgt. Das Ausgangsmaterial liegt in feiner Pulverschicht vor, deren Partikel durch den Laser verschmolzen werden und dadurch das Pulver Schicht für Schicht miteinander verbunden wird. Demnach werden über das SLS räumliche Strukturen aus einem pulverförmigen Ausgangsstoff hergestellt. Dabei ist die Verarbeitung von verschiedenen kunststoffähnlichen Materialien möglich. SLS verschmilzt selektiv Pulvermaterialien wie Nylon, Elastomere, Alumide oder Polyamide.

Ein weiteres im Stand der Technik bekanntes Verfahren ist das "Fused Deposition Modeling" (FDM). Das FDM wird auch als Schmelzschichtungsverfahren bezeichnet. Denn beim FDM wird ein 3D-Objekt schichtweise aus einem schmelzfähigen Kunststoff hergestellt. Dabei wird mit Acrylnitril-Butadien-Styrol (ABS) gearbeitet, d.h. einem Kunststoff, der sich besonders für Einsatzobjekte mit hoher Beanspruchung eignet.

Ferner ist die Polygrafie bekannt (auch bekannt als Polyjet- oder Inkjet-Verfahren). Die Polygraphie ist ein 3D-Druckverfahren, bei dem ein Photopolymer Schicht für Schicht auf ein Substrat aufgebracht und anschließend mittels UV-Licht ausgehärtet wird. Hierbei wird das zu druckende Objekt durch einen Druckkopf aufgebaut, der ähnlich wie der Druckkopf eines Tintenstrahldruckers arbeitet. Damit es möglich ist, Überhänge an den Objekten zu drucken, wird Stützmaterial mitgedruckt. Deshalb verfügen diese 3D-Drucker über zwei oder auch mehr Druckköpfe. Abwechselnd wird dann Bau- und Stützmaterial verdruckt. Schicht für Schicht werden die Konturen des Objekts auf der Bauplattform aufgespritzt. Als Material wird ein haltbares und formbeständiges Photopolymer (Kunstharz) verwendet. Das zunächst im Drucker flüssige Material verhärtet sich, wenn Schicht für Schicht nacheinander mit UV-Licht belichtet wird. Die Polygrafie ermöglicht die Herstellung detaillierter Objekte mit einem hohen Detailgrad und einer glatten Oberfläche.

Darüberhinaus ist das sogenannte "3D Printing" (3DP) bekannt. Beim 3D Printing entsteht ein 3D-Objekt durch ein gipsartiges Pulver, das stufenweise dünnen Schichten verteilt wird. Mithilfe eines Binders haftet die jeweils nächste Schicht an der darunterliegenden Schicht. Mehrere hundert oder gar tausend Schichten bilden auf diese Weise ein gewünschtes 3D-Objekt.

Im Stand der Technik ist zudem bekannt, biologisches Material über ein biologisches Druckverfahren (engl. "bioprinting") herzustellen. Das "bioprinting" ist das einzige Verfahren, welches biologisches Material in einem dreidimensionalen Aufbau (kurz: 3D-Aufbau) bereitstellen kann (z.B. ganze biologische Zellen). Das "bioprinting" hat den Vorteil, dass es bei einer Temperatur unter 40°C betrieben werden kann, sodass eine hitzebedingte Schädigung von biologischem Material ausgeschlossen werden kann.

Die im "bioprinting" verwendbaren Ausgangssubstanzen können nicht immer identisch sein mit den Substanzen, welche von lebenden Organismen in der Natur für den Aufbau von biologischem Material verwendet werden. Dies kann ein Problem für den Aufbau darstellen bzw. einen naturgemäßen Aufbau verhindern. Es kann jedoch auch ein Vorteil sein: Ein Aufbau von biologischem Material mit Reinsubstanzen und in einer exakt definierbaren Umgebung erlaubt die Bereitstellung von biologischem Material in einer höheren Reinheit und in einer höheren strukturellen Homogenität als das entsprechende, in der Natur vorkommende Material. In der Natur erfolgt der Aufbau von biologischem Material regelmäßig durch eine Aneinanderbindung einzelner, kleiner Moleküle (z.B. Nukleotide, Aminosäuren oder Lipide) zu einem größeren Gebilde (z.B. Lipid-Doppelschicht) oder zu einem größeren Molekül (z.B. DNA oder Proteine). Ein weiterer Vorteil des "bioprinting" kann sich dadurch ergeben, dass das biologische Material nicht wie in der Natur, sondern Schicht für Schicht aufgebaut wird, sodass die durch den Schichtaufbau bewirkten mechanischen Eigenschaften eines über das "bioprinting" bereitgestellten Materials gezielt eingestellt werden können.

Die EP 2 836 558 A1 offenbart eine abbaubare Mischung aus biologischen Komponenten, wobei die Mischung einen Proteinkleber aus wenigstens einem Protein, Naturfasern, ein hygroskopisches Mineral, Wasser und optional ein Additiv enthält. Die abbaubare Mischung kann zu einem dreidimensionalen abbaubaren Material gedruckt werden.

Die US 2017/0239388 A1 offenbart ein Verfahren zum Erzeugen von 3D-Gerüsten unter Verwendung von nanoskaligen Fasern. Das Verfahren umfasst unter anderem eine Ablagerung und Ausrichtung einer Vielzahl von elektrogesponnenen Faserschichten auf einem Substrat, einem Aufbringen einer lichtempfindlichen biomedizinischen Polymerflüssigkeit auf jede Faserschicht, einer Ablagerung und Querausrichtung einer Vielzahl von elektrogesponnenen Faserschichten auf dem Substrat, einem Halten der Polymerflüssigkeit an Ort und Stelle unter Verwendung der kreuzweise ausgerichteten Faserschichten und einem Aushärten der Polymerflüssigkeit auf jeder Faserschicht unter Verwendung von UV-Licht.

Enzyme können die kovalente Verbindung von zwei kleinen Molekülen (Bausteine) zu einem größeren Molekül katalysieren. Wie alle Katalysatoren liegen Enzyme nach der Katalyse eines solchen Prozesses wieder in der Ausgangsform vor. Unter zahlreichen in einer biologischen Umgebung vorkommenden Stoffen katalysieren Enzyme regelmäßig nur eine bestimmte Reaktion (hohe Reaktionsspezifität) und regelmäßig wird diese Reaktion nur für ein oder mehrere bestimmte Substrate katalysiert (hohe Substratspezifität).

Tijore, A. et al. (Biofabrication, Bd. 10, Nr. 2, 025003, 2018, S. 1-14) offenbaren ein über einen 3D-Druck hergestelltes Gelatin-Hydrogel, das durch die Wirkung von mikrobieller Transglutaminase (mTgase) quervernetzt wurde und Mikrokanäle aufweist, welche einerseits die Differenzierung von hMSC-Zellen zu Cardiomyocyten begünstigen und andererseits die Aktivität und Organisation von differenzierten, rhytmisch schlagenden Cardiomyocyten verbessern.

Die EP 2 836 558 B1 offenbart ein abbaubares Material aus biologischen Komponenten sowie ein Verfahren zur Herstellung eines Formteils aus einem abbaubaren Material.

Aufgrund dieser Eigenschaften wäre wünschenswert, wenn Enzyme über einen 3D-Druck auf einem Substrat deponiert werden könnten. Bislang bekannte 3D-Druckverfahren arbeiten jedoch bei Temperaturen, die so hoch sind, dass die Enzyme denaturieren würden und ihre Enzymaktivität verlieren würden. Ferner war es bislang nicht möglich, auf einfache und schnelle Art und Weise ein dreidimensionales biologisches Material bereitzustellen, das bessere chemische, biologische und/oder mechanische Eigenschaften aufweist als bereits bekanntes (vergleichbares) biologisches Material.

Ausgehend hiervon war es die Aufgabe der vorliegenden Erfindung, eine Vorrichtung und ein Verfahren bereitzustellen, mit der/dem auf einfache und schnelle Art und Weise ein dreidimensionales biologisches Material gedruckt werden kann, dessen Eigenschaften präziser als mit bekannten Vorrichtungen bzw. Verfahren gesteuert werden können und sich somit 3D-Drucke mit verbesserte (biologische, chemische und mechanische) Eigenschaften gegenüber bekannten, vergleichbaren biologischen Materialien, erzielen lassen.

Die Aufgabe wird gelöst durch den 3D-Drucker mit den Merkmalen von Anspruch 1, das Verfahren zum 3D-Druck eines biologischen Materials gemäß Anspruch 7, den Extruder, den Spritzgießer oder die Formpresse gemäß Anspruch 13, das Verfahren zur Extrusion, zum Formpressen oder zum Spritzguss gemäß Anspruch 14 und das biologische Material gemäß Anspruch 15. Die abhängigen Ansprüche zeigen vorteilhafte Weiterbildungen auf.

Erfindungsgemäß wird ein 3D-Drucker bereitgestellt, enthaltend
a) eine Kammer, optional enthaltend ein biologisches Material;
b) eine Dosiereinheit zum Inkontaktbringen von mindestens einem Enzym mit einem in der Kammer befindlichen Material;
c) eine Steuerungseinheit;
dadurch gekennzeichnet, dass die Dosiereinheit mindestens ein Enzym enthält, das dazu geeignet ist, eine Reaktion zu katalysieren, die dazu geeignet ist, eine kovalente (chemische) Verbindung von biologischem Material zu bewirken, und die Steuerungseinheit dazu konfiguriert ist, das Inkontaktbringen des mindestens einen Enzyms mit einem in der Kammer befindlichen biologischen Material zu veranlassen.

Ein wesentlicher Vorteil des erfindungsgemäßen 3D-Druckers besteht darin, dass ein Inkontaktbringen des Enzyms mit dem biologischen Material von der Steuerungseinheit veranlasst wird, d.h. der Zeitpunkt des Inkontaktbringens kann gesteuert werden und damit der Beginn der kovalenten Verbindungsreaktion reguliert werden. Grundsätzlich beginnt die kovalente Verbindungsreaktion nach dem Inkontaktbringen von Enzym mit biologischem Material. Es kann jedoch noch eine weitere Stufe der Regulation enthalten sein. Beispielsweise kann das Enzym ein Enzym sein, dass erst ab einem bestimmten Temperaturbereich die kovalente Verbindungsreaktion katalysiert. Die nötige Reaktionswärme kann veranlasst werden durch ein (lokales) Aufheizen der Kammer des 3D-Druckers über eine Heizung des 3D-Druckers und/oder ein (lokales) Bestrahlen der Kammer über eine Einstrahleinheit für elektromagnetische Strahlung (z.B. ein IR-Laser). Ferner sind Enzyme denkbar, die durch elektromagnetische Strahlung schaltbar sind und sich durch Einwirkung von elektromagnetischer Strahlung (z.B. einen VIS-Laser) aktivieren lassen. Es wird somit über die Steuerungseinheit ein Einfluss auf den Startzeitpunkt der Reaktion erlaubt, der eine präzisere Kontrolle über die Eigenschaften des über den 3D-Drucker gedruckten biologischen Materials (3D-Druck) ermöglicht.

Ein weiterer Vorteil des erfindungsgemäßen Druckers ist, dass die Einwirkdauer des Enzyms auf das Substrat gezielt gesteuert werden kann und somit die Eigenschaften (z.B. mechanischen Eigenschaften) eines zu druckenden biologischen Materials gezielt beeinflusst werden kann. Den Startpunkt der Einwirkzeit legt die Steuerungseinheit fest, indem sie das Inkontaktbringen von Enzym und biologischem Material zu einem gewünschten Zeitpunkt veranlasst. Der Endpunkt der Einwirkzeit kann ebenso durch die Steuerungseinheit veranlasst werden. Beispielsweise kann die biologische Wirkung des Enzyms durch Hitzeeinwirkung zerstört werden. Die Hitzeeinwirkung kann veranlasst werden durch ein (lokales) Aufheizen der Kammer des 3D-Druckers über eine Heizung des 3D-Druckers und/oder ein (lokales) Bestrahlen der Kammer über eine Einstrahleinheit für elektromagnetische Strahlung (z.B. ein IR-Laser). Über diese Maßnahme kann ein Abstoppen der Wirkung des Enzyms erreicht werden, wodurch die Wirkzeit des Enzyms verglichen mit der Wirkzeit des Enzyms in der Natur verringert oder verlängert werden kann. Dadurch kann der Vernetzungsgrad einzelner Komponenten des biologischen Materials und damit dessen finaler Härtegrad gegenüber seinem in der Natur vorkommenden Vorbild gezielt gesenkt oder angehoben werden.

Ferner ist ein Vorteil des erfindungsgemäßen 3D-Druckers, dass der Einwirkort des Enzyms auf das Substrat gezielt gesteuert werden kann und somit die Eigenschaften (z.B. mechanischen Eigenschaften) eines zu druckenden biologischen Materials gezielt beeinflusst werden kann. Beispielsweise kann durch die Dosiereinheit des Druckers das Enzym an Stellen auf dem Substrat aufgebracht werden, die sich von den in der Natur vorkommenden Wirkstellen des jeweiligen Enzyms unterscheiden. Über diese Maßnahme können Strukturen geschaffen werden, die eine höhere oder niedrigere strukturelle Ordnung und damit zumindest bereichsweise eine höhere oder niedrigere Festigkeit als ihr jeweiliges Vorbild aus der Natur aufweisen (z.B. künstlich verfestigtes Perlmutt oder künstlich verfestigte Lignocellulose).

Der 3D-Drucker kann biologisches Material bereitstellen, das eine hohe Reinheit aufweist, insbesondere eine höhere Reinheit als das entsprechende in der Natur vorkommende biologische Material. Beispielsweise kann komplett ausgeschlossen werden, dass das biologische Material Verunreinigungen aufweist, die sonst üblicherweise bei einer Isolation des biologischen Materials aus seiner natürlichen Umgebung auftreten (z.B. Allergene, Toxine etc.). Wegen der hohen Substrat- bzw. Reaktionsspezifität von Enzymen können auch nicht-homogene bzw. mit anderen Substanzen vermischte oder verunreinigte biologische Materialien spezifisch kovalent verknüpft werden.

Der beschriebene 3D-Drucker ermöglicht den Aufbau einer Wertschöpfungszellularität. Diese ermöglicht bei Kombination mit einer Enzymproduktion in Nachbarschaft zum 3D-Drucker, z.B. in einem Fermenter, eine Wertschöpfungskette. Dabei wird das Werkzeug, d.h. das Enzym, bei Bedarf, z.B. wenn ein Füllstandssensor des 3D-Druckers einen Bedarf an Enzym meldet, nachproduziert und für den weiteren 3D-Druck bereitgestellt. Zwischen Fermentation und 3D-Druck liegt ein Zwischenschritt, der automatisiert geführt werden kann: Abtrennung und Aufreingung des mindestens einen für den 3D-Druck eingesetzten Enzyms.

Der 3D-Drucker kann dadurch gekennzeichnet sein, dass die Kammer biologisches Material enthält, das sich über Enzymkatalyse kovalent verknüpfen lässt, wobei das biologische Material bevorzugt Moleküle enthält, die ausgewählt sind aus der Gruppe bestehend aus Kohlenhydrate, Proteine, Lipide, Lignin, Phenole und Mischungen hiervon, optional Monomere, Oligomere und/oder Polymere dieser Moleküle, wobei die Moleküle bevorzugt ausgewählt sind aus der Gruppe bestehend aus Cellulose, Stärke, Chitin bzw. Chitosan, Proteine, Lipide, Lignin, Phenole und Mischungen hiervon, optional ausgewählt aus der Gruppe bestehend aus Proteine, Phenole und Mischungen hiervon. Bevorzugt enthält das biologische Material Chitin, Chitosan und/oder Lignin, oder besteht daraus.

Ferner kann das biologische Material ein anorganisches Material enthalten, bevorzugt ein anorganisches Salz, das sich zur Kristallbildung eignet, besonders bevorzugt ein Calciumsalz, insbesondere Calciumphosphat und/oder Calciumcarbonat.

Zudem kann das biologische Material Moleküle enthalten, welche die kovalenten Verbindung des biologischen Materials begünstigen, bevorzugt Moleküle ausgewählt aus der Gruppe bestehend aus Enzymsubstraten, Enzym-Cofaktoren, chemisch aktivierte Moleküle und Mischungen hiervon, wobei die Enzymsubstrate insbesondere ausgewählt sind aus Kohlenhydraten, Proteinen, Peptiden, Nukleinsäuren, Lipiden, Phenolen, Phenylpropanoiden und Mischungen hiervon, optional Monomere, Oligomere und/oder Polymere dieser Moleküle und/oder der Enzym-Cofaktor insbesondere Lysyl-Tyrosyl-Chinon ist und/oder die Enzymsubstrate insbesondere chemisch aktivierte Moleküle sind .

Das biologische Material kann in fester, flüssiger, gelförmiger oder knetfähiger Form vorliegen, bevorzugt in Form eines festen Pulvers oder in Form fester Filamente.

In einer vorteilhaften Ausgestaltungsform enthält die Kammer ein biologisches Material, das Protein in einer Menge von > 0 Gew.-% bis 9 Gew.-%, bevorzugt 0,5 Gew.-% bis 8 Gew.-%, besonders bevorzugt 1 Gew.-% bis 6 Gew.-%, insbesondere 1,5 Gew.-% bis 4 Gew.-%, Protein, bezogen auf die Gesamtmenge an biologischem Material, aufweist.

Die Kammer kann ferner einen Temperatursensor zur Messung einer Temperatur in der Kammer enthalten. Dieser ist vorteilhaft, wenn die Kammer auf die für die Aktivität des Enzyms optimale Temperatur temperierbar ist.

Die Kammer kann temperierbar sein, bevorzugt auf eine Temperatur im Bereich von 30 °C bis 60°C, besonders bevorzugt auf eine Temperatur im Bereich von 30 bis 50°C, besonders bevorzugt auf einer Temperatur im Bereich von 30 °C bis 45 °C, wobei die Kammer insbesondere eine Heizung enthält.

Zudem kann die Kammer einen Drucksensor zur Messung eines Drucks in der Kammer enthalten.

Die Kammer kann unter Druck setzbar sein, bevorzugt auf einen Druck im Bereich von 0,1 bis 20 bar, bevorzugt 0,5 bis 15 bar, besonders bevorzugt 1 bis 10 bar, wobei die Kammer insbesondere mit einer Druckluftquelle verbunden ist.

Darüberhinaus kann die Kammer einen Luftfeuchtigkeitssensor zur Messung einer Luftfeuchtigkeit in der Kammer enthalten.

Zudem kann die Kammer einen Materialfeuchtigkeitssensor zur Messung einer Feuchtigkeit des Materials in der Kammer enthalten, bevorzugt einen auf einer Stromwiderstandsmessung basierender Materialfeuchtigkeitssensor.

Die Kammer kann auf eine auf eine bestimmte Feuchtigkeit einstellbar sein, wobei die Kammer insbesondere mit einer Wasserquelle oder Wasserdampfquelle verbunden ist.

Ferner kann die Kammer entfeuchtbar sein, wobei die Kammer insbesondere mit einem Kondensator und/oder einem Trockenmittel verbunden ist.

Der 3D-Drucker kann dadurch gekennzeichnet sein, dass die Dosiereinheit in x-Richtung, y-Richtung und in z-Richtung beweglich ausgestaltet ist, bevorzugt über eine kartesische Kinematik, besonders bevorzugt in Richtung und/oder entlang eines Bodens der Kammer beweglich ausgestaltet ist.

Die Dosiereinheit kann mit einer elektrischen Spannungsquelle zur Abgabe von elektrischer Spannung an die Dosiereinheit verbunden sein und/oder mit einer Ultraschallquelle verbunden sein, wobei die Spannungsquelle und/oder Ultraschallquelle bevorzugt dazu geeignet ist/sind, eine Freisetzung des mindestens einen Enzyms aus biologischen Zellen zu bewirken, bevorzugt über eine Lyse der biologischen Zellen.

Ferner kann die Dosiereinheit fluidisch mit einem Reservoir verbunden sein, das dazu geeignet ist, eine Substanz (z.B. ein Enzym) in die Dosiereinheit zu geben, wobei die Substanz bevorzugt dazu geeignet ist, eine Produktion und/oder Freisetzung des mindestens einen Enzyms in biologischen Zellen zu bewirken, bevorzugt über einen Transport der Substanz aus den biologischen Zellen und/oder einer Lyse der biologischen Zellen.

Zudem kann die Dosiereinheit in Richtung der Kammer einen Sprühkopf aufweisen.

In einer bevorzugten Ausgestaltungsform enthält die Dosiereinheit einen Temperatursensor zur Messung einer Temperatur in der Dosiereinheit.

Die Dosiereinheit kann temperierbar sein, bevorzugt auf eine Temperatur von weniger als 45°C, besonders bevorzugt auf eine Temperatur im Bereich von 1 bis 20°C, bevorzugt im Bereich von 2 bis 10°C, besonders bevorzugt im Bereich von 3°C bis 5°C, wobei die Dosiereinheit insbesondere eine Kühlung enthält.

Die Dosiereinheit kann einen Drucksensor zur Messung eines Drucks in der Dosiereinheit enthalten.

Ferner kann die Dosiereinheit unter Druck setzbar sein, bevorzugt auf einen Druck im Bereich von 0,1 bis 20 bar, bevorzugt 0,5 bis 15 bar, besonders bevorzugt 1 bis 10 bar, wobei die Dosiereinheit insbesondere mit einer Druckluftquelle verbunden ist.

Zudem kann die Dosiereinheit einen Luftfeuchtigkeitssensor zur Messung einer Luftfeuchtigkeit in der Dosiereinheit enthalten.

Die Dosiereinheit kann ferner auf eine bestimmte Feuchtigkeit einstellbar sein, wobei die Dosiereinheit insbesondere mit einer Wasserquelle und/oder Wasserdampfquelle verbunden ist.

Darüberhinaus kann die Dosiereinheit ein pH-Meter aufweisen.

Die Dosiereinheit ist in einer bevorzugten Ausgestaltungsform mit einem UV-Vis-Spektrometer verbunden, das zur Messung einer UV-Vis-Absorption eines im Innenraum der Dosiereinheit befindlichen Mediums geeignet ist, wobei das UV-Spektrometer bevorzugt dazu konfiguriert ist, eine Absorption bei einer Wellenlänge im Bereich von 240 nm bis 750 nm, bevorzugt 280 bis 700 nm, zu messen.

Die Dosiereinheit kann einen Füllstandssensor aufweisen, der zur Messung eines Volumens einer Enzymlösung in der Dosiereinheit geeignet ist.

Die Dosiereinheit kann Moleküle enthalten, welche die kovalenten Verbindung des biologischen Materials begünstigen, bevorzugt ein Molekül ausgewählt aus der Gruppe bestehend aus Enzymsubstraten, Enzym-Cofaktoren, chemisch aktivierte Moleküle und Mischungen hiervon, wobei die Enzymsubstrate insbesondere ausgewählt sind aus der Gruppe bestehend aus Kohlenhydraten, Proteinen, Peptiden, Nukleinsäuren, Lipiden, Phenolen, Phenylpropanoiden und Mischungen hiervon, optional Monomere, Oligomere und/oder Polymere dieser Moleküle, und/oder der Enzym-Cofaktor insbesondere Lysyl-Tyrosyl-Chinon ist und/oder die Enzymsubstrate insbesondere chemisch aktivierte Moleküle sind.

In einer bevorzugten Ausgestaltungsform enthält die Dosiereinheit keine Naturfasern, bevorzugt kein biologisches Material, das kovalent durch eine von dem mindestens einen Enzym katalysiere Reaktion verbunden wird. Vorteil dieser Ausgestaltungsform ist, dass das Enzym noch nicht in der Dosiereinheit eine Verbindung von biologischem Material bewirken kann, da dieses nicht in der Dosiereinheit vorhanden ist. Mit anderen Worten wird sichergestellt, dass die kovalente Verbindung des biologischen Materials erst nach Inkontaktbringen des Enzyms der Dosiereinheit mit dem biologischen Material der Kammer erfolgt.

In einer weiteren bevorzugten Ausgestaltungsform enthält die Dosiereinheit kein hygroskopisches Mineral, bevorzugt kein hygroskopisches Material. Der Vorteil an dieser Ausgestaltungsform ist, dass in der Dosiereinheit pulverförmiges Enzym eingesetzt werden kann ohne dass ein Risiko besteht, dass durch hygroskopisches Material Wasser gezogen wird, das pulverförmige Enzym verklumpt und eine Öffnung der Dosiereinheit verstopft wird.

Die Dosiereinheit kann einen öffenbaren Verschluss in Richtung Kammer aufweisen. Bevorzugt ist die Steuerungseinheit dazu konfiguriert, eine Öffnung des öffenbaren Verschluss zu veranlassen, besonders bevorzugt basierend auf einem Befehl eines Benutzers.

Das mindestens eine Enzym kann in fester oder gelöster Form vorliegen, bevorzugt in Form eines festen Pulvers oder gelöst in einer wässrigen Pufferlösung oder einem wässrigen Fermentationsmedium.

Ferner kann das mindestens eine Enzym von festen Filamenten enthalten sein und/oder in festen Filamenten immobilisiert sein.

Darüberhinaus kann das Enzym in biologischen Zellen enthalten sein und/oder in der Zellmembran von biologischen Zellen enthalten sein, wobei die biologischen Zellen bevorzugt ausgewählt sind aus der Gruppe bestehend aus prokaryotischen Zellen, eukaryotischen Zelle und Mischungen hiervon, besonders bevorzugt ausgewählt aus der Gruppe bestehend aus Bakterienzellen, Archaeenzellen, pilzlichen, pflanzlichen und tierischen Zellen (z.B. menschlichen Zellen) und Mischungen hiervon.

Zudem kann das Enzym in einem Innenraum der Dosiereinheit vorhanden sein und/oder an einer der Kammer zugewandten äußeren Wand der Dosiereinheit immobilisiert sein.

Ferner kann das Enzym in der Kammer vorliegen.

In einer bevorzugten Ausgestaltungsform ist das Enzym ausgewählt ist aus der Gruppe bestehend aus Laccasen, Tyrosinase, Peroxidasen, Phenoloxidasen Aminosäureoxidasen, Transglutaminasen und Mischungen hiervon. Das Enzym kann auch ausgewählt sein aus der Gruppe bestehend aus Aminooxidase, Transglutaminase, Laccase, Tyrosinase, Peroxidase, Phenoloxidase und Mischungen hiervon.Die Steuerungseinheit des 3D-Druckers kann ferner dazu konfiguriert sein, eine Temperatur der Kammer und/oder Dosiereinheit zu regeln, bevorzugt auf eine Temperatur im Bereich von 30 °C bis 60°C, besonders bevorzugt von 30 bis 50°C, besonders bevorzugt auf einer Temperatur im Bereich von 30 °C bis 45 °C, für die Kammer und/oder auf eine Temperatur im Bereich von 1 bis 20°C, bevorzugt von 2 bis 10°C, besonders bevorzugt von 3°C bis 5°C, für die Dosiereinheit, insbesondere über Steuerung einer Heizung und/oder Kühlung der Kammer und/oder Dosiereinheit.

Zudem kann die Steuerungseinheit dazu konfiguriert sein, einen Druck in der Kammer und/oder Dosiereinheit zu regeln, bevorzugt auf einen Druck im Bereich von 0,1 bis 20 bar, bevorzugt 0,5 bis 15 bar, besonders bevorzugt 1 bis 10 bar, insbesondere über Steuerung einer mit der Kammer und/oder Dosiereinheit verbundenen Druckluftquelle.

Darüberhinaus kann die Steuerungseinheit dazu konfiguriert sein, eine Feuchtigkeit in der Kammer und/oder Dosiereinheit zu regeln, insbesondere über eine mit der Kammer und/oder Dosiereinheit verbundenen Wasserquelle oder Wasserdampfquelle.

Ferner kann die Steuerungseinheit dazu konfiguriert sein, den pH-Wert eines Mediums zu bestimmen, in dem sich das mindestens eine Enzym befindet (z.B. eine wässrige Pufferlösung als Medium), insbesondere über ein pH-Meter der Dosiereinheit befindet.

Die Steuerungseinheit kann zudem dazu konfiguriert sein, eine Konzentration des mindestens einen Enzym in einem Medium zu bestimmen, in dem sich das mindestens eine Enzym befindet, insbesondere über ein UV-Spektrometer, das mit der Dosiereinheit verbunden ist.

Darüberhinaus kann die Steuerungseinheit dazu konfiguriert sein, ein Volumen des mindestens einen Enzyms in der Dosiereinheit zu bestimmen, insbesondere über einen Füllstandssensor, der von der Dosiereinheit enthalten ist.

In einer bevorzugten Ausgestaltungsform ist die Steuerungseinheit dazu konfiguriert, eine Einwirkzeit des mindestens einen Enzyms auf ein in der Kammer befindliches biologisches Material zu bestimmen.

Darüberhinaus kann die Steuerungseinheit dazu konfiguriert sein, mehrere Schichten von biologischem Material über Enzymeinwirkung miteinander zu verbinden.

Zudem kann die Steuerungseinheit dazu konfiguriert sein, das Inkontaktbringen des mindestens einen Enzyms mit einem in der Kammer befindlichen (biologischen) Material auf eine Art und Weise zu veranlassen, dass die Gesamtmenge von Enzym in einem Bereich von 0,001 Gew.-% bis <0,01 Gew.-%, bevorzugt 0,002 Gew.-% bis 0,009 Gew.-%, besonders bevorzugt 0,003 Gew.-% bis 0,008 Gew.-%, insbesondere 0,004 Gew.-% bis 0,007 Gew.-%, bezogen auf die Gesamtmenge von Enzym und biologischem Material in der Kammer, liegt.

Der 3D-Drucker kann dadurch gekennzeichnet sein, dass er eine Einstrahleinheit enthält, die dazu geeignet ist, elektromagnetische Strahlung in die Kammer zu strahlen, wobei die Einstrahleinheit bevorzugt dazu geeignet ist,
a) Infrarotlicht in die Kammer zu strahlen, wobei die Einstrahleinheit besonders bevorzugt ausgewählt ist aus der Gruppe bestehend aus IR-Lampe, IR-Laser und Kombinationen hiervon; und/oder
b) UV-Licht in die Kammer zu strahlen, wobei die Einstrahleinheit besonders bevorzugt ausgewählt ist aus der Gruppe bestehend aus UV-Lampe, UV-Laser und Kombinationen hiervon;
wobei die Steuerungseinheit insbesondere dazu konfiguriert ist, eine bestimmte Zeit nach dem Inkontaktbringen des mindestens einen Enzyms mit dem in der Kammer befindlichen biologischen Material mit der Einstrahleinheit zumindest bereichsweise auf das biologische Material zu strahlen, sodass das mindestens eine Enzym aktiviert oder deaktiviert wird.

Ferner kann der 3D-Drucker dadurch gekennzeichnet sein, dass er mit einem Fermenter und/oder einer Quelle von biologischem Material verbunden ist. Diese Ausgestaltungsform hat den Vorteil, dass der 3D-Drucker permanent und ohne Unterbrechung mit Enzym und/oder biologischem Material versorgt werden kann und somit für sehr lange Zeit bzw. sogar kontinuierlich betrieben werden kann.

Ferner wird ein Verfahren zum 3D-Druck eines biologischen Materials vorgestellt, umfassend die Schritte
a) Bereitstellen eines biologischen Materials in einer Kammer eines 3D-Druckers, die zur Aufnahme von biologischem Material geeignet ist;
b) Bereitstellen einer Dosiereinheit, die zum Inkontaktbringen von mindestens einem Enzym mit einem in der Kammer befindlichen Material geeignet ist;
c) Bereitstellung einer Steuerungseinheit in dem 3D-Drucker;
dadurch gekennzeichnet, dass die Dosiereinheit mit mindestens einem Enzym ausgestattet wird, das dazu geeignet ist, eine Reaktion zu katalysieren, die dazu geeignet ist, eine kovalente Verbindung von biologischem Material zu bewirken, und die Steuerungseinheit veranlasst, dass das mindestens eine Enzym mit dem in der Kammer befindlichen biologischen Material in Kontakt gebracht wird.

Das Verfahren erlaubt den 3D-Druck eines biologischen Materials mit den oben im Zusammenhang mit dem 3D-Drucker genannten Vorteilen.

Das Verfahren kann dadurch gekennzeichnet sein, dass in der Kammer biologisches Material bereitgestellt wird, das sich über Enzymkatalyse kovalent verknüpfen lässt, wobei das biologische Material bevorzugt Moleküle enthält, die ausgewählt sind aus der Gruppe bestehend aus Kohlenhydrate, Proteine, Lipide, Lignin, Phenole und Mischungen hiervon, optional Monomere, Oligomere und/oder Polymere dieser Moleküle, wobei die Moleküle bevorzugt ausgewählt sind aus der Gruppe bestehend aus Cellulose, Stärke, Chitin bzw. Chitosan, Proteine, Lipide, Lignin, Phenole und Mischungen hiervon, optional ausgewählt aus der Gruppe bestehend aus Proteine, Phenole und Mischungen hiervon. Bevorzugt enthält das biologische Material Chitin, Chitosan und/oder Lignin, oder besteht daraus.

Das in dem Verfahren eingesetzte biologische Material kann ein anorganisches Material enthalten, bevorzugt ein anorganisches Salz, das sich zur Kristallbildung eignet, besonders bevorzugt ein Calciumsalz, insbesondere Calciumphosphat und/oder Calciumcarbonat.

Ferner kann das in dem Verfahren eingesetzte biologische Material Moleküle enthalten, welche die kovalenten Verbindung des biologischen Materials begünstigen, bevorzugt ein Molekül ausgewählt aus der Gruppe bestehend aus Enzymsubstraten, Enzym-Cofaktoren, chemisch aktivierte Moleküle und Mischungen hiervon, wobei die Enzymsubstrate insbesondere ausgewählt sind aus Kohlenhydraten, Proteinen, Peptiden, Nukleinsäuren, Lipiden, Phenolen, Phenylpropanoiden und Mischungen hiervon, optional Monomere, Oligomere und/oder Polymere dieser Moleküle und/oder der Enzym-Cofaktor insbesondere Lysyl-Tyrosyl-Chinon ist und/oder die Enzymsubstrate insbesondere chemisch aktivierte Moleküle sind.

Das biologische Material kann in fester, flüssiger, gelförmiger oder knetfähiger Form vorliegen, bevorzugt in Form eines festen Pulvers oder in Form fester Filamente.

In einer vorteilhaften Ausführungsform des Verfahrens wird in der Kammer ein biologisches Material bereitgestellt, das Protein in einer Menge von > 0 Gew.-% bis 9 Gew.-%, bevorzugt 0,5 Gew.-% bis 8 Gew.-%, besonders bevorzugt 1 Gew.-% bis 6 Gew.-%, insbesondere 1,5 Gew.-% bis 4 Gew.-%, Protein, bezogen auf die Gesamtmenge an biologischem Material, aufweist.

Das Verfahren kann dadurch gekennzeichnet sein, dass die Kammer einen Temperatursensor zur Messung einer Temperatur in der Kammer enthält.

Ferner kann die Kammer in dem Verfahren temperiert werden, bevorzugt auf eine Temperatur im Bereich von 30 °C bis 60°C, besonders bevorzugt auf eine Temperatur im Bereich von 30 bis 50°C, besonders bevorzugt auf einer Temperatur im Bereich von 30 °C bis 45 °C, insbesondere über eine Heizung, welche die Kammer enthält.

Darüberhinaus kann die Kammer einen Drucksensor zur Messung eines Drucks in der Kammer enthalten.

Die Kammer kann in dem Verfahren unter Druck gesetzt werden, bevorzugt auf einen Druck im Bereich von 0,1 bis 20 bar, bevorzugt 0,5 bis 15 bar, besonders bevorzugt 1 bis 10 bar, insbesondere über eine Druckluftquelle, mit der die Kammer verbunden ist.

Zudem ist es möglich, dass die Kammer einen Luftfeuchtigkeitssensor zur Messung einer Luftfeuchtigkeit in der Kammer enthält.

Ferner kann die Kammer einen Materialfeuchtigkeitssensor zur Messung einer Feuchtigkeit des Materials in der Kammer enthalten, bevorzugt einen auf einer Stromwiderstandsmessung basierenden Materialfeuchtigkeitssensor.

In dem Verfahren kann die Kammer auf eine bestimmte Feuchtigkeit eingestellt werden, insbesondere über eine Wasserquelle oder Wasserdampfquelle, die mit der die Kammer verbunden ist.

In einer bevorzugten Ausführungsform des Verfahrens wird die Dosiereinheit, in x-Richtung, y-Richtung und in z-Richtung bewegt, über eine kartesische Kinematik, besonders bevorzugt in Richtung und/oder entlang eines Bodens der Kammer (d.h. auf das biologische Material zu und seitlich entlang des biologischen Materials).

Die Dosiereinheit kann ferner mit einer elektrischen Spannungsquelle zur Abgabe von elektrischer Spannung an die Dosiereinheit verbunden werden und/oder mit einer Ultraschallquelle verbunden werden, wobei die Spannungsquelle und/oder die Ultraschallquelle bevorzugt dazu verwendet wird, eine Freisetzung des mindestens einen Enzyms aus biologischen Zellen zu bewirken, bevorzugt über eine Lyse der biologischen Zellen.

In dem Verfahren kann die Dosiereinheit fluidisch mit einem Reservoir verbunden werden, das dazu geeignet ist, eine Substanz in die Dosiereinheit zu geben, wobei die Substanz bevorzugt dazu geeignet ist, eine Produktion und/oder Freisetzung des mindestens einen Enzyms in biologischen Zellen zu bewirken, bevorzugt über einen Transport der Substanz aus den biologischen Zellen und/oder einer Lyse der biologischen Zellen.

Zudem kann die Dosiereinheit in Richtung der Kammer mit einem Sprühkopf ausgestattet werden.

Ferner kann die Dosiereinheit mit einem Temperatursensor zur Messung einer Temperatur in der Dosiereinheit ausgestattet werden.

In einer bevorzugten Ausführungsform ist die Dosiereinheit temperierbar oder wird temperiert, bevorzugt auf eine Temperatur im Bereich von 1°C bis 20°C, bevorzugt von 2°C bis 10°C, besonders bevorzugt von 3°C bis 5°C, wobei die Dosiereinheit insbesondere eine Kühlung enthält.

Die Dosiereinheit kann in dem Verfahren mit einem Drucksensor zur Messung eines Drucks in der Dosiereinheit ausgestattet werden.

Ferner kann die Dosiereinheit in dem Verfahren unter Druck setzbar sein oder gesetzt werden, bevorzugt auf einen Druck im Bereich von 0,1 bis 20 bar, bevorzugt 0,5 bis 15 bar, besonders bevorzugt 1 bis 10 bar, insbesondere über Druckluft aus einer Druckluftquelle, mit der die Dosiereinheit verbunden ist oder wird.

Zudem kann die Dosiereinheit mit einem Luftfeuchtigkeitssensor zur Messung einer Luftfeuchtigkeit in der Dosiereinheit ausgestattet werden.

Darüberhinaus kann die Dosiereinheit auf eine bestimmte Feuchtigkeit einstellbar sein oder eingestellt werden, wobei die Dosiereinheit insbesondere über Wasser und/oder Wasserdampf aus einer Wasserquelle und/oder Wasserdampfquelle, mit der die Dosiereinheit verbunden ist oder wird.

Die Dosiereinheit kann mit einem pH-Meter ausgestattet werden.

Zudem kann die Dosiereinheit in dem Verfahren mit einem UV-Vis-Spektrometer verbunden werden und eine Messung einer UV-Vis-Absorption eines im Innenraum der Dosiereinheit befindlichen Mediums durchgeführt werden, wobei bevorzugt eine Absorption bei einer Wellenlänge im Bereich von 240 nm bis 750 nm, bevorzugt 280 bis 700 nm, gemessen wird.

Die Dosiereinheit kann ferner mit einem Füllstandssensor ausgestattet werden, der zur Messung eines Volumens einer Enzymlösung in der Dosiereinheit geeignet ist.

Es ist auch möglich, die Dosiereinheit in dem Verfahren mit Molekülen auszustatten, welche die kovalenten Verbindung des biologischen Materials begünstigen, bevorzugt Moleküle ausgewählt aus der Gruppe bestehend aus Enzymsubstraten, Enzym-Cofaktoren, chemisch aktivierte Moleküle und Mischungen hiervon, wobei die Enzymsubstrate insbesondere ausgewählt sind aus der Gruppe bestehend aus Kohlenhydraten, Proteinen, Peptiden, Nukleinsäuren, Lipiden, Phenolen, Phenylpropanoiden und Mischungen hiervon, optional Monomere, Oligomere und/oder Polymere dieser Moleküle, und/oder der Enzym-Cofaktor insbesondere Lysyl-Tyrosyl-Chinon ist und/oder die Enzymsubstrate insbesondere chemisch aktivierten Moleküle sind.

In einer bevorzugten Ausführungsform enthält die Dosierkammer keine Naturfasern, bevorzugt kein biologisches Material enthält, das kovalent durch eine von dem mindestens einen Enzym katalysiere Reaktion verbunden wird.

In einer weiteren bevorzugten Ausführungsfrom enthält die Dosierkammer kein hygroskopisches Mineral, besonders bevorzugt kein hygroskopisches Material.

Die Dosiereinheit kann einen öffenbaren Verschluss in Richtung Kammer aufweisen. Bevorzugt wird die Steuerungseinheit veranlasst, den öffenbaren Verschluss zu öffnen, besonders bevorzugt basierend auf einem Befehl eines Benutzers.

Das mindestens eine in dem Verfahren eingesetzte Enzym kann in fester oder gelöster Form bereitgestellt werden, bevorzugt in Form eines festen Pulvers oder gelöst in einer wässrigen Pufferlösung oder einem wässrigen Fermentatiosmedium.

Das mindestens eine Enzym kann ferner in festen Filemanten enthalten sein und/oder in festen Filamenten immobilisiert sein.

Zudem ist es möglich, dass das Enzym in biologischen Zellen enthalten bereitgestellt wird und/oder in der Zellmembran von biologischen Zellen bereitgestellt wird, wobei die biologischen Zellen bevorzugt ausgewählt sind aus der Gruppe bestehend aus prokaryotischen Zellen, eukaryotischen Zelle und Mischungen hiervon, besonders bevorzugt ausgewählt aus der Gruppe bestehend aus Bakterienzellen, Archaeenzellen, pilzlichen, pflanzlichen oder tierischen Zellen (z.B. menschlichen Zellen) und Mischungen hiervon.

Das Enzym kann in einem Innenraum der Dosiereinheit bereitgestellt werden und/oder an einer der Kammer zugewandten äußeren Wand der Dosiereinheit immobilisiert bereitgestellt werden.

Das Enzym kann in der Kammer bereitgestellt werden bzw. in der Kammer vorliegen.

In einer bevorzugten Ausführungsform ist das Enzym ausgewählt aus der Gruppe bestehend aus Laccasen, Tyrosinase, Peroxidasen, Phenoloxidasen, Aminosäureoxidasen,Transglutaminasen, und Mischungen hiervon. Das Enzym kann auch ausgewählt sein aus der Gruppe bestehend aus Aminooxidase, Transglutaminase, Laccase, Tyrosinase, Peroxidase, Phenoloxidase und Mischungen hiervon.

In dem Verfahren kann über die Steuerungseinheit eine Temperatur der Kammer und/oder Dosiereinheit geregelt werden, bevorzugt auf eine Temperatur im Bereich von 30 °C bis 60°C, besonders bevorzugt von 30 bis 50°C, besonders bevorzugt auf einer Temperatur im Bereich von 30 °C bis 45 °C, für die Kammer und/oder auf eine Temperatur im Bereich von 1 bis 20°C, bevorzugt von 2 bis 10°C, besonders bevorzugt von 3°C bis 5°C, für die Dosiereinheit, insbesondere über Steuerung einer Heizung und/oder Kühlung der Kammer und/oder Dosiereinheit.

Ferner kann in dem Verfahren über die Steuerungseinheit ein Druck in der Kammer und/oder Dosiereinheit geregelt werden, bevorzugt auf einen Druck im Bereich von 0,1 bis 20 bar, bevorzugt 0,5 bis 15 bar, besonders bevorzugt 1 bis 10 bar, insbesondere über Steuerung einer mit der Kammer und/oder Dosiereinheit verbundenen Druckluftquelle.

Zudem ist es möglich, dass in dem Verfahren eine Feuchtigkeit in der Kammer und/oder Dosiereinheit geregelt wird, insbesondere über eine mit der Kammer und/oder Dosiereinheit verbundenen Wasserquelle oder Wasserdampfquelle.

Ferner kann in dem Verfahren über die Steuerungseinheit der pH-Wert eines Medium bestimmt werden, in dem sich das mindestens eine Enzym befindet, insbesondere über ein pH-Meter der Dosiereinheit.

Darüberhinaus kann in dem Verfahren eine Konzentration des mindestens einen Enzyms in einem Medium bestimmt werden, in dem sich das mindestens eine Enzym befindet, insbesondere über ein UV-Spektrometer, das mit der Dosiereinheit verbunden ist.

In einer bevorzugten Ausführungsform wird das Volumen mindestens einer Enzymlösung in der Dosiereinheit bestimmt, insbesondere über einen Füllstandssensor, der von der Dosiereinheit enthalten ist.

Ferner kann in dem Verfahren eine Einwirkzeit des mindestens einen Enzyms auf ein in der Kammer befindliches biologisches Material bestimmt werden.

In einer bevorzugten Ausführungsform veranlasst die Steuerungseinheit, dass das mindestens eine Enzym mit dem in der Kammer befindlichen biologischen Material auf eine Art und Weise in Kontakt gebracht wird, dass die Gesamtmenge von Enzym in einem Bereich von 0,001 Gew.-% bis <0,01 Gew.-%, bevorzugt 0,002 Gew.-% bis 0,009 Gew.-%, besonders bevorzugt 0,003 Gew.-% bis 0,008 Gew.-%, insbesondere 0,004 Gew.-% bis 0,007 Gew.-%, bezogen auf die Gesamtmenge von Enzym und biologischem Material in der Kammer, liegt.

Das Verfahren kann dadurch gekennzeichnet sein, dass der 3D-Drucker mit einer Einstrahleinheit ausgestattet wird, die dazu geeignet ist, elektromagnetische Strahlung in die Kammer zu strahlen, wobei die Einstrahleinheit bevorzugt dazu geeignet ist,
a) Infrarotlicht in die Kammer zu strahlen, wobei die Einstrahleinheit besonders bevorzugt ausgewählt ist aus der Gruppe bestehend aus IR-Lampe, IR-Laser und Kombinationen hiervon; und/oder
b) UV-Licht in die Kammer zu strahlen, wobei die Einstrahleinheit besonders bevorzugt ausgewählt ist aus der Gruppe bestehend aus UV-Lampe, UV-Laser und Kombinationen hiervon;
wobei eine bestimmte Zeit nach dem Inkontaktbringen des mindestens einen Enzyms mit dem in der Kammer befindlichen biologischen Material mit der Einstrahleinheit zumindest bereichsweise auf das biologische Material gestrahlt wird, sodass das mindestens eine Enzym aktiviert oder deaktiviert wird. Durch diese Maßnahme kann die Einwirkzeit des Enzyms auf das biologische Material gezielt gesteuert werden und gezielt auf die Eigenschaften des erzeugten biologischen Materials Einfluss genommen werden.

In einer bevorzugten Ausführungsform wird in dem Verfahren der 3D-Drucker mit einem Fermenter und/oder einer Quelle von biologischem Material verbunden. Diese Ausführungsform hat den Vorteil, dass der 3D-Drucker permanent und ohne Unterbrechung mit Enzym und/oder biologischem Material versorgt werden kann und somit für sehr lange Zeit bzw. sogar kontinuierlich betrieben werden kann.

In einer bevorzugten Ausführungsform des Verfahrens werden mehrere Schichten von biologischem Material über Enzymeinwirkung miteinander verbunden.

Es wird ferner ein Extruder, ein Spritzgießer oder eine Formpresse bereitgestellt, enthaltend
a) eine Kammer zur Aufnahme und Extrusion von einem Material, optional enthaltend ein biologisches Material;
b) eine Dosiereinheit zum Inkontaktbringen von mindestens einem Enzym mit einem in der Kammer befindlichen Material; und
c) eine Steuerungseinheit;
dadurch gekennzeichnet, dass die Dosiereinheit mindestens ein Enzym enthält, das dazu geeignet ist, eine Reaktion zu katalysieren, die dazu geeignet ist, eine kovalente Verbindung von biologischem Material zu bewirken, und die Steuerungseinheit dazu konfiguriert ist, das Inkontaktbringen des mindestens einen Enzyms mit einem in der Kammer befindlichen biologischen Material auf eine Art und Weise zu veranlassen, dass die Gesamtmenge von Enzym in einem Bereich von 0,001 Gew.-% bis <0,01 Gew.-%, bevorzugt 0,002 Gew.-% bis 0,009 Gew.-%, besonders bevorzugt 0,003 Gew.-% bis 0,008 Gew.-%, insbesondere 0,004 Gew.-% bis 0,007 Gew.-%, bezogen auf die Gesamtmenge von Enzym und biologischem Material in der Kammer, liegt.

Der Vorteil des Extruders, des Spritzgießers bzw. der Formpresse ist, dass die kovalente Verbindung von biologischem Material auf sehr sparsame Art und Weise erfolgt, da die Steuerungseinheit dazu konfiguriert ist, nur sehr geringe Enzymmengen zu dem biologischen Material zuzugeben, um die kovalente Verbindung zu erreichen. Da die Herstellung von der benötigten Art von Enzymen sehr kostspielig ist, ist die Bereitstellung von kovalent verbundenem biologischen Material über den erfindungsgemäßen Extruder, den erfindungsgemäßen Spritzgießer bzw. die erfindungsgemäße Formpresse sehr ökonomisch.

In einer vorteilhaften Ausgestaltungsform enthält die Kammer ein biologisches Material, das Protein in einer Menge von > 0 Gew.-% bis 9 Gew.-%, bevorzugt 0,5 Gew.-% bis 8 Gew.-%, besonders bevorzugt 1 Gew.-% bis 6 Gew.-%, insbesondere 1,5 Gew.-% bis 4 Gew.-%, Protein, bezogen auf die Gesamtmenge an biologischem Material, aufweist. Da Proteine in der Fertigung eingespart werden können, die besser für die menschliche/tierische Ernährung verwendet werden, ist die Bereitstellung von kovalent verbundenem biologischen Material über diese Ausgestaltungsform ökonomischer.

Die Kammer kann temperierbar sein, bevorzugt auf eine Temperatur im Bereich von 30 °C bis 60°C, besonders bevorzugt auf eine Temperatur im Bereich von 30 bis 50°C, besonders bevorzugt auf einer Temperatur im Bereich von 30 °C bis 45 °C, wobei die Kammer insbesondere eine Heizung enthält. Da die Temperierung der Kammer auf Temperaturen von mehr als 60 °C unnötig viel Heizenergie verbraucht, ist die Bereitstellung von kovalent verbundenem biologischen Material über diese Ausgestaltungsform ökonomischer und ökologischer als bei Extrudern, Spritzgießern oder Formpressen, die dazu konfiguriert sind, die Kammer auf über 60 °C zu temperieren.

Der erfindungsgemäße Extruder, der erfindungsgemäße Spritzgießer bzw. die erfindungsgemäße Formpresse kann mindestens ein (optional jedes) Merkmal aufweisen, das oben im Zusammenhang mit dem erfindungsgemäßen 3D-Drucker (als bevorzugtes Merkmal) genannt wurde.

Zudem wird ein Verfahren zur Extrusion, zum Formpressen oder zum Spritzguss eines biologischen Materials vorgestellt, umfassend die Schritte
a) Bereitstellen eines biologischen Materials in einer Kammer eines Extruders, einer Formpresse oder eines Spritzgießers, wobei die Kammer zur Aufnahme von biologischem Material geeignet ist;
b) Bereitstellen einer Dosiereinheit, die zum Inkontaktbringen von mindestens einem Enzym mit dem in der Kammer befindlichen biologischen Material geeignet ist; und
c) Bereitstellung einer Steuerungseinheit (optional in dem Extruder, in der Formpresse oder in dem Spritzgießer);
dadurch gekennzeichnet, dass die Dosiereinheit mit mindestens einem Enzym ausgestattet wird, das dazu geeignet ist, eine Reaktion zu katalysieren, die dazu geeignet ist, eine kovalente Verbindung von biologischem Material zu bewirken, und die Steuerungseinheit veranlasst, dass das mindestens eine Enzym mit dem in der Kammer befindlichen biologischen Material auf eine Art und Weise in Kontakt gebracht wird, dass die Gesamtmenge von Enzym in einem Bereich von 0,001 Gew.-% bis <0,01 Gew.-%, bevorzugt 0,002 Gew.-% bis 0,009 Gew.-%, besonders bevorzugt 0,003 Gew.-% bis 0,008 Gew.-%, insbesondere 0,004 Gew.-% bis 0,007 Gew.-%, bezogen auf die Gesamtmenge von Enzym und biologischem Material in der Kammer, liegt.

Das erfindungsgemäße Verfahren zur Extrusion, zum Formpressen oder zum Spritzguss eines biologischen Materials hat den Vorteil, dass das die kovalente Verbindung von biologischem Material auf sehr sparsame Art und Weise erfolgt, da die Steuerungseinheit veranlasst, nur sehr geringe Enzymmengen zu dem biologischen Material zuzugeben, um die kovalente Verbindung zu erreichen. Da die Herstellung von der benötigten Art von Enzymen sehr kostspielig ist, ist die Bereitstellung von kovalent verbundenem biologischen Material über das erfindungsgemäße Verfahren sehr ökonomisch.

In einer vorteilhaften Ausführungsform des Verfahrens wird in der Kammer ein biologisches Material bereitgestellt, das Protein in einer Menge von > 0 Gew.-% bis 9 Gew.-%, bevorzugt 0,5 Gew.-% bis 8 Gew.-%, besonders bevorzugt 1 Gew.-% bis 6 Gew.-%, insbesondere 1,5 Gew.-% bis 4 Gew.-%, Protein, bezogen auf die Gesamtmenge an biologischem Material, aufweist. Da der Einsatz von proteinhaltigem biologischen Material kostspieliger ist als ein Einsatz von proteinarmen Material, ist die Bereitstellung von kovalent verbundenem biologischen Material über diese Ausführungsform ökonomischer.

Die Kammer kann auf eine Temperatur im Bereich von 30 °C bis 60°C, besonders bevorzugt auf eine Temperatur im Bereich von 30 bis 50°C, besonders bevorzugt auf einer Temperatur im Bereich von 30 °C bis 45 °C, temperiert werden, insbesondere über eine Heizung der Kammer. Da die Temperierung der Kammer auf Temperaturen von mehr als 60 °C unnötig viel Heizenergie verbraucht, ist die Bereitstellung von kovalent verbundenem biologischen Material über diese Ausführungsform ökonomischer und ökologischer als Verfahren, bei denen eine Temperierung der Kammer auf über 60 °C vorgesehen ist.

Das erfindungsgemäße Verfahren zur Extrusion, zum Formpressen oder zum Spritzguss eines biologischen Materials kann mindestens ein (optional jedes) Merkmal aufweisen, das oben im Zusammenhang mit dem erfindungsgemäßen Verfahren zum 3D-Druck eines biologischen Materials (als bevorzugtes Merkmal) genannt wurde.

Es wird ferner ein biologisches Material bereitgestellt, das über das Verfahren zum 3D-Druck eines biologischen Materials, bei dem mehrere Schichten von biologischem Material über Enzymeinwirkung miteinander verbunden wurden, hergestellt wurde. Das bereitgestellte biologische Material weist somit zwangsweise alle Eigenschaften auf, die sich zwangsweise aus der Durchführung des (jeweiligen) erfindungsgemäßen Verfahrens ergeben. Das erfindungsgemäße biologische Material ist dadurch gekennzeichnet, dass das biologische Material einen Schichtaufbau aufweist, wobei sich zwischen den Schichten des verbundenen biologischen Materials das Enzym befindet, das dazu geeignet ist, eine Reaktion zu katalysieren, die dazu geeignet ist, eine kovalente Verbindung von biologischem Material zu bewirken, wobei die Gesamtmenge des Enzyms, das dazu geeignet ist, eine Reaktion zu katalysieren, die dazu geeignet ist, eine kovalente Verbindung von biologischem Material zu bewirken, in einem Bereich von 0,001 Gew.-% bis <0,01 Gew.-%, bezogen auf die Gesamtmenge von Enzym und biologischem Material, liegt.

Erfindungsgemäß weist das biologische Material einen Schichtaufbau auf, wobei sich zwischen den Schichten des verbundenen biologischen Materials Enzym befindet. Ein solches biologisches Material ist im Stand der Technik unbekannt. Das zwischen den Schichten des biologischen Materials befindliche Enzym kann noch aktiv sein oder inaktiv sein (beispielsweise über die oben genannte Einstrahleinheit deaktiviert worden sein). Ein Vorteil eines noch aktiven Enzyms in dem biologischen Material kann darin liegen, dass im Falle einer Beschädigung des biologischen Materials eine Kontaktierung der Schadstelle mit Wasser bzw. wässrigem Lösung eine Enzymtätigkeit hervorrufen kann, wodurch die im biologischen Material aufgetretenen Beschädigungen (z.B. Risse) wieder verschlossen werden könnten (Selbstreparatur bzw. Selbstheilung durch Aufbau neuer kovalenter Verbindungen im biologischen Material).

Anhand der nachfolgenden Figuren und Beispiele soll der erfindungsgemäße Gegenstand näher erläutert werden, ohne diesen auf die hier dargestellten, spezifischen Ausführungsformen einschränken zu wollen.

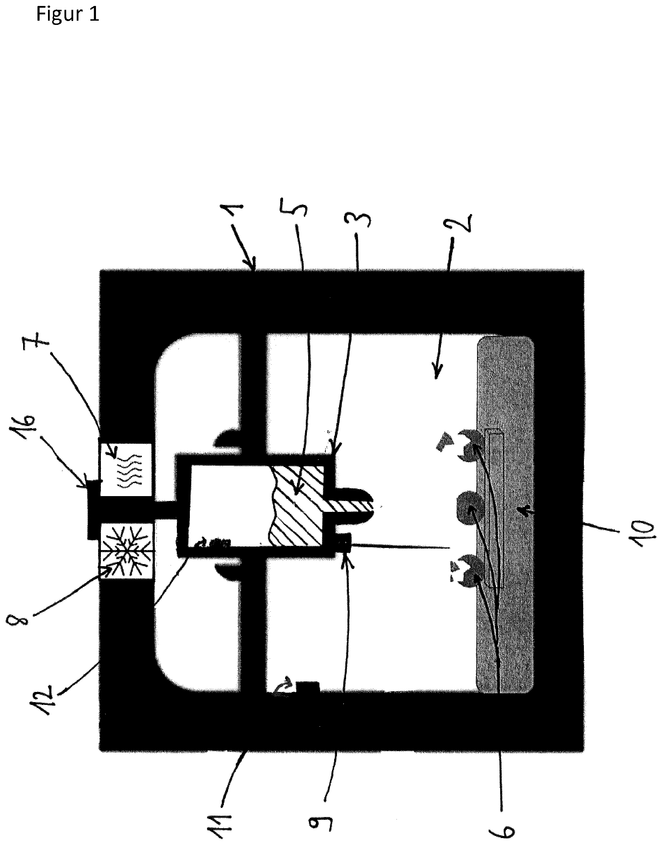

Figur 1 zeigt ein Beispiel eines erfindungsgemäßen 3D-Druckers 1. Der 3D-Drucker weist eine Kammer 2 und eine Dosiereinheit 3 auf. Die Dosiereinheit 3 enthält das Enzym 5, das eine Reaktion katalysiert, die eine kovalente Verbindung von biologischem Material bewirkt. In diesem Fall liegt das Enzym 5 in einer wässrigen Lösung gelöst vor. Auf dem Boden 10 der Kammer 2 befindet sich das biologische Material 6, das über die Wirkung des Enzyms 5 kovalent verbunden werden kann. Das biologische Material 6 ist über die Öffnung 16 des 3D-Druckers in die Kammer 2 befördert worden. Zusätzlich enthält dieser 3D-Drucker eine Einstrahleinheit 9 für elektromagnetische Strahlung (hier: ein IR-Laser). Ferner weist dieser 3D-Drucker eine Heizung 7 und eine Kühlung 8 auf, deren Heizwirkung und Kühlwirkung von einem Temperatursensor 11 in der Kammer 2 erfasst wird. Auch die Dosiereinheit 3 weist einen Temperatursensor 12 auf, der zur Überwachung der Temperatur in der Dosiereinheit 3 dient.

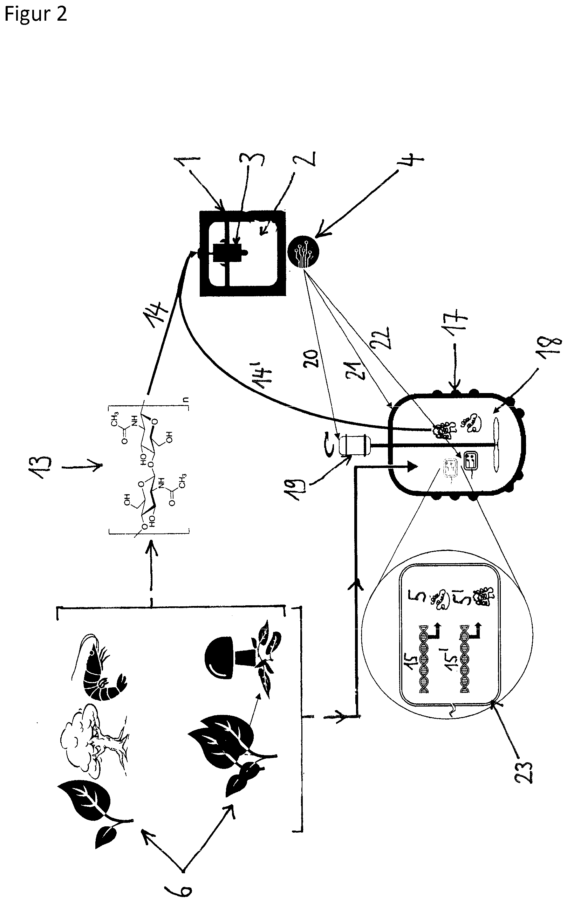

Figur 2 zeigt ein weiteres Beispiel eines erfindungsgemäßen 3D-Druckers 1. Dieser 3D-Drucker 1 weist eine Kammer 2 und eine Dosiereinheit 3 auf und ist mit einem Fermenter 17 und einer Quelle für biologisches Material 6 verbunden (nicht gezeigt), sodass ein (kontinuierlicher) Transport 14, 14' von biologischem Material 6 und Enzym aus dem Fermenter 17 in den 3D-Drucker transportiert werden kann. Es ist ein Ausschnitt 13 einer molekularen Struktur eines beispielhaften biologischen Materials 6 dargestellt. In den biologischen Zellen 23 im Innenraum 18 des Fermenters 17 wird über einen Induktor 15 die Produktion des Enzyms 5 gestartet und über einen weiteren Induktor 15' die Produktion eines weiteren Enzyms 5' gestartet. Eine Rührvorrichtung 19 des Fermenters 17 sorgt für eine Durchmischung der biologischen Zellen 23. Die Steuerungseinheit 4 des 3D-Druckers übernimmt hierbei die Steuerung 20 der Rührvorrichtung 19, die Steuerung 21 einer Heizung des Fermenters 17 und die Steuerung 22 einer Zugabe von Induktor 15, 15'.

### Beispiel 1 - 3D-Drucker mit pulverisiertem Biomaterial

Die Grundstruktur des 3D-Druckers ist die eines 3D Pulverdruckers (SLM) oder eines Filamentdruckers (FDM).

Die Kammer des 3D-Druckers wird mit einem pulverisiertem Biomaterial gefüllt.

Die Dosiereinheit (z.B. ein Tröpfchenerzeuger) enthält einen wässrigen Puffer, der das mindestens eine Enzym (optional auch kovalent verbindbare Monomeren oder kurze Ketten) enthält. Optional weist die Dosiereinheit eine Kühlung auf.

Über die Dosiereinheit kann dieser Puffer mit Enzym in Flüssigkeitstropfen auf das Pulverbett des biologischen Materials abgegeben werden. Der Puffer dringt ein bzw. wird durch Kapillarkräfte in die Zwischenräume des biologischen Materials aufgenommen. Durch das Enzym wird eine Reaktion katalysiert, die eine kovalente Verknüpfung des biologischen Materials bewirkt (optional eine zusätzliche Verknüpfung zu den in der Dosiereinheit enthaltenen Monomeren oder kurze Ketten).

Falls die Dosiereinheit eine Kühlung aufweist, kann über die Kühlung die Lebensdauer der Enzyme verlängert werden. Enthält die Dosiereinheit Monomere oder kurze Ketten, die untereinander durch die vom Enzym katalyisierte Reaktion kovalent verbunden werden können, kann durch die Senkung der Temperatur der Dosiereinheit diese Reaktion unterdrückt werden. Es kann somit verhindert werden, dass sich Monomere und kurze Ketten miteinander verbinden, bevor sie mit dem biologischen Material in der Kammer in Kontakt treten. Nach dem Verlassen der gekühlten Dosiereinheit kann die Enzymaktivität durch ein Erhitzen der Kammer des 3D-Druckers (z.B. über ein Heizelement) gesteigert werden (z.B. auf die optimale Reaktionstemperatur des Enzyms).

Die Enzyme verbleiben im kovalent verbundenen Substrat und können ggf. wieder durch Zutritt von Flüssigkeit (z.B. Puffer) reaktiviert werden. Sind in dem biologischen Material noch unvernetzte Substrat-Moleküle vorhanden, kann durch die Enzyme ein Verschluss eines feinen Risses von innen heraus katalysiert werden (Selbstreparatur).

Der 3D-Drucker kann einen Laser (z.B. IR-Laser) enthalten, mit dem an bestimmten Stellen des in der Kammer befindlichen biologischen Materials ein lokaler Wärmeeintrag durchgeführt werden kann. Durch diese Maßnahme kann lokal eine Enzymaktivität gesteigert werden, aber durch ein starkes Erhitzen eine Enzymaktivität (durch Enzymdenaturierung) aber auch ganz und irreversibel gestoppt werden. Es kann somit auf einen Verbindungsgrad (Vernetzungsgrad) im biologischen Material Einfluss genommen werden.

### Beispiel 2 - Kontinuierlich arbeitender 3D-Drucker mit verbundener Enzymquelle

In dieser Ausgestaltungsform wird das Enzym zunächst in einem Fermentationsprozess hergestellt. Dort werden Bakterien oder Hefen kultiviert, die dann das Enzym ins Medium sezernieren, das permanent entnommen und automatisiert aufgereinigt werden kann oder das Enzym befindet sich in oder auf den Zellen (Biomasse), wobei das Enzym (auf den Zellen) einer Aufreinigung zugeführt wird. Im ersten Fall wird das Enzym im Fermentationsmedium bereitgestellt, im letzteren Fall steht am Ende ein gereinigtes Enzym bereit.

Das Enzym kann über eine Fluidleitung direkt von dem Fermenter oder direkt von einem Reinigungsschritt der Dosiereinheit des 3D-Druckers zugeführt werden. In diesem Fall ist die Dosiereinheit des 3D-Druckers fluidisch mit einem Fermenter und/oder einer Vorrichtung zur Aufreinigung des Enzyms verbunden.

Der Fermenter kann in einem kontinuierlichen Prozess arbeiten. Wenn der Enzymdrucker feststellt, dass die vorhandene Enzymmenge bald zu Ende gehen wird, wird der in Warteposition befindliche Fermenter die optimalen Randbedingungen einstellen (Substratzugabe, Temperaturerhöhung, etc.) um das Wachstum der Mikroorganismen hochzufahren. Besteht eine fluidische Verbindung zwischen Enzymquelle (Fermenter) und der Dosiereineit des 3D-Druckers, kann auch der 3D-Drucker kontinuierlich arbeiten.

### Bezugszeichenliste:

- 1:: 3D-Drucker;
- 2:: Kammer des 3D-Druckers;
- 3:: Dosiereinheit des 3D-Druckers;
- 4:: Steuerungseinheit des 3D-Druckers;
- 5:: Enzym, das eine Reaktion katalysiert, die eine kovalente Verbindung von biologischem Material bewirkt;
- 5':: weiteres Enzym, das eine Reaktion katalysiert, die eine kovalente Verbindung von biologischem Material bewirkt;
- 6:: biologisches Material (z.B. in Pulverform), das sich über eine Katalysereaktion des Enzyms 5 kovalent verknüpfen lässt;
- 7:: Heizung (der Kammer);
- 8:: Kühlung (der Kammer);
- 9:: Einstrahleinheit (z.B. ein IR-Laser);
- 10:: Boden der Kammer;
- 11:: Temperatursensor (der Kammer);
- 12:: Temperatursensor (der Dosiereinheit);
- 13:: Ausschnitt einer molekularen Struktur des biologischen Materials;
- 14:: Transport von biologischem Material 6 in die Kammer des 3D-Druckers;
- 14':: Transport von Enzym 5, 5' in die Kammer des 3D-Druckers;
- 15:: Induktor des Enzyms 5;
- 15':: Induktor des weiteren Enzyms 5';
- 16:: Öffnung des 3D-Druckers;
- 17:: Fermenter zur Herstellung des Enzyms 5 bzw. weiteren Enzyms 5';
- 18:: Innenraum des Fermenters;
- 19:: Rührvorrichtung des Fermenters;
- 20:: Steuerung der Rührvorrichtung über die Steuerungseinheit des 3D-Druckers;
- 21:: Steuerung einer Heizung des Fermenters über die Steuerungseinheit des 3D-Druckers;
- 22:: Steuerung einer Zugabe von Induktor der Enzyme 5, 5' über die Steuerungseinheit des 3D-Druckers;
- 23:: biologische Zellen zur Herstellung von Enzym 5, 5'.

## Patentansprüche

1. 3D-Drucker (1), enthaltend
a) eine Kammer (2), optional enthaltend ein biologisches Material (6);
b) eine Dosiereinheit (3) zum Inkontaktbringen von mindestens einem Enzym (5) mit einem in der Kammer (2) befindlichen Material; und
c) eine Steuerungseinheit (4);
**dadurch gekennzeichnet, dass** die Dosiereinheit (3) mindestens ein Enzym (5) enthält, das dazu geeignet ist, eine Reaktion zu katalysieren, die dazu geeignet ist, eine kovalente Verbindung von biologischem Material zu bewirken, und die Steuerungseinheit (4) dazu konfiguriert ist, das Inkontaktbringen des mindestens einen Enzyms (5) mit einem in der Kammer (2) befindlichen biologischen Material zu veranlassen.

2. 3D-Drucker (1) gemäß Anspruch 1, **dadurch gekennzeichnet, dass** die Kammer (2)
i) einen Temperatursensor (11) zur Messung einer Temperatur in der Kammer (2) enthält; und/oder
ii) temperierbar ist, bevorzugt auf eine Temperatur im Bereich von 30 bis 60°C, besonders bevorzugt auf eine Temperatur im Bereich von 30 bis 50°C temperierbar ist, wobei die Kammer (2) insbesondere eine Heizung (7) enthält; und/oder
iii) einen Drucksensor zur Messung eines Drucks in der Kammer (2) enthält; und/oder
iv) unter Druck setzbar ist, bevorzugt auf einen Druck im Bereich von 0,1 bis 20 bar, bevorzugt 0,5 bis 15 bar, besonders bevorzugt 1 bis 10 bar, wobei die Kammer (2) insbesondere mit einer Druckluftquelle verbunden ist; und/oder
v) einen Luftfeuchtigkeitssensor zur Messung einer Luftfeuchtigkeit in der Kammer (2) enthält; und/oder
vi) einen Materialfeuchtigkeitssensor zur Messung einer Feuchtigkeit des Materials in der Kammer (2) enthält, bevorzugt einen auf einer Stromwiderstandsmessung basierender Materialfeuchtigkeitssensor; und/oder
vii) auf eine bestimmte Feuchtigkeit einstellbar ist, wobei die Kammer (2) insbesondere mit einer Wasserquelle oder Wasserdampfquelle verbunden ist; und/oder
viii) entfeuchtbar ist, wobei die Kammer (2) insbesondere mit einem Kondensator und/oder einem Trockenmittel verbunden ist.

3. 3D-Drucker (1) gemäß einem der vorhergehenden Ansprüche, **dadurch gekennzeichnet, dass** die Dosiereinheit (3)
i) in x-Richtung, y-Richtung und in z-Richtung beweglich ausgestaltet ist, bevorzugt über eine kartesische Kinematik, besonders bevorzugt in Richtung eines Bodens (10) der Kammer (2) beweglich ausgestaltet ist; und/oder
ii) mit einer elektrischen Spannungsquelle zur Abgabe von elektrischer Spannung an die Dosiereinheit (3) verbunden ist und/oder mit einer Ultraschallquelle verbunden ist, wobei die Spannungsquelle und/oder Ultraschallquelle bevorzugt dazu geeignet ist/sind, eine Freisetzung des mindestens einen Enzyms (5) in biologischen Zellen zu bewirken, bevorzugt über eine Lyse der biologischen Zellen; und/oder
iii) fluidisch mit einem Reservoir verbunden ist, das dazu geeignet ist, eine Substanz in die Dosiereinheit (3) zu geben, wobei die Substanz bevorzugt dazu geeignet ist, eine Produktion und/oder Freisetzung des mindestens einen Enzyms (5) in biologischen Zellen zu bewirken, bevorzugt über einen Transport der Substanz aus den biologischen Zellen und/oder einer Lyse der biologischen Zellen; und/oder
iv) in Richtung der Kammer (2) einen Sprühkopf aufweist; und/oder
v) einen Temperatursensor zur Messung einer Temperatur in der Dosiereinheit (3) enthält; und/oder
vi) temperierbar ist, bevorzugt auf eine Temperatur von 1 bis 20°C, bevorzugt im Bereich von 2 bis 10°C, besonders bevorzugt im Bereich von 3°C bis 5°C, temperierbar ist, wobei die Dosiereinheit (3) insbesondere eine Kühlung enthält; und/oder
vii) einen Drucksensor zur Messung eines Drucks in der Dosiereinheit (3) enthält; und/oder
viii) unter Druck setzbar ist, bevorzugt auf einen Druck im Bereich von 0,1 bis 20 bar, bevorzugt 0,5 bis 15 bar, besonders bevorzugt 1 bis 10 bar, wobei die Dosiereinheit (3) insbesondere mit einer Druckluftquelle verbunden ist; und/oder
ix) einen Luftfeuchtigkeitssensor zur Messung einer Luftfeuchtigkeit in der Dosiereinheit (3) enthält; und/oder
x) auf eine bestimmte Feuchtigkeit einstellbar ist, wobei die Dosiereinheit (3) insbesondere mit einer Wasserquelle und/oder Wasserdampfquelle verbunden ist; und/oder
xi) ein pH-Meter aufweist; und/oder
xii) mit einem UV-Vis-Spektrometer verbunden ist, das zur Messung einer Licht-Absorption eines im Innenraum der Dosiereinheit (3) befindlichen Mediums geeignet ist, wobei das UV-Vis-Spektrometer bevorzugt dazu konfiguriert ist, eine Absorption bei einer Wellenlänge im Bereich von 240 bis 750 nm, bevorzugt 280 nm bis 700 nm, zu messen; und/oder
xiii) einen Füllstandssensor aufweist, der zur Messung eines Volumens einer Enzymlösung in der Dosiereinheit (3) geeignet ist; und/oder
xiv) Moleküle enthält, welche die kovalente Verbindung des biologischen Materials begünstigen, bevorzugt ein Molekül ausgewählt aus der Gruppe bestehend aus Enzymsubstraten, Enzym-Cofaktoren, chemisch aktivierten Molekülen und Mischungen hiervon, wobei die Enzymsubstrate insbesondere ausgewählt sind aus der Gruppe bestehend aus Kohlenhydraten, Proteinen, Peptiden, Nukleinsäuren, Lipiden, Phenolen, Phenylpropanoiden und Mischungen hiervon, optional Monomere, Oligomere und/oder Polymere dieser Moleküle und/oder der Enzym-Cofaktor insbesondere Lysyl-Tyrosyl-Chinon ist und/oder die Enzymsubstrate insbesondere chemisch aktivierten Moleküle sind; und/oder
xv) keine Naturfasern enthält, bevorzugt kein biologisches Material (6) enthält, das kovalent durch eine von dem mindestens einen Enzym (5) katalysiere Reaktion verbunden wird; und/oder
xvi) kein hygroskopisches Mineral enthält, bevorzugt kein hygroskopisches Material enthält; und/oder
xvii) einen öffenbaren Verschluss in Richtung Kammer (2) aufweist, wobei bevorzugt die Steuerungseinheit (4) dazu konfiguriert ist, eine Öffnung des öffenbaren Verschluss zu veranlassen, besonders bevorzugt basierend auf einem Befehl eines Benutzers.

4. 3D-Drucker (1) gemäß einem der vorhergehenden Ansprüche, **dadurch gekennzeichnet, dass** das mindestens eine Enzym (5)
i) in gelöster oder fester Form vorliegt, bevorzugt gelöst in einer wässrigen Pufferlösung oder einem wässrigen Fermentationsmedium oder in Form eines festen Pulvers ; und/oder
ii) von festen Filamenten enthalten ist und/oder in festen Filamenten immobilisiert ist; und/oder
iii) in biologischen Zellen enthalten ist und/oder in der Zellmembran von biologischen Zellen enthalten ist, wobei die biologischen Zellen bevorzugt ausgewählt sind aus der Gruppe bestehend aus prokaryotischen Zellen, eukaryotischen Zelle und Mischungen hiervon, besonders bevorzugt ausgewählt aus der Gruppe bestehend aus Bakterienzellen, Archaeenzellen, pilzlichen, pflanzlichen und tierischen Zellen und Mischungen hiervon; und/oder
iv) in einem Innenraum der Dosiereinheit (3) vorhanden ist; und/oder
v) in der Kammer (2) vorliegt; und/oder
vi) an einer der Kammer (2) zugewandten äußeren Wand der Dosiereinheit (3) immobilisiert ist; und/oder
vii) ausgewählt ist aus der Gruppe bestehend aus Laccasen, Tyrosinase, Peroxidasen, Phenoloxidasen Aminosäureoxidasen, Transglutaminasen, und Mischungen hiervon.

5. 3D-Drucker (1) gemäß einem der vorhergehenden Ansprüche, **dadurch gekennzeichnet, dass** die Steuerungseinheit (4) dazu konfiguriert ist,
i) eine Temperatur der Kammer (2) und/oder Dosiereinheit (3) zu regeln, bevorzugt auf eine Temperatur im Bereich von 30 bis 60°C, besonders bevorzugt von 30 bis 50°C, für die Kammer (2) und/oder auf eine Temperatur im Bereich von 1 bis 20°C, bevorzugt von 2 bis 10°C, besonders bevorzugt von 3 bis 5°C für die Dosiereinheit (3), insbesondere über Steuerung einer Heizung (7) und/oder Kühlung (8) der Kammer (2) und/oder Dosiereinheit (3); und/oder
ii) einen Druck in der Kammer (2) und/oder Dosiereinheit (3) zu regeln, bevorzugt auf einen Druck im Bereich von 0,1 bis 20 bar, bevorzugt 0,5 bis 15 bar, besonders bevorzugt 1 bis 10 bar, insbesondere über Steuerung einer mit der Kammer (2) und/oder Dosiereinheit (3) verbundenen Druckluftquelle; und/oder
iii) eine Feuchtigkeit in der Kammer (2) und/oder Dosiereinheit (3) zu regeln, insbesondere über eine mit der Kammer (2) und/oder Dosiereinheit (3) verbundenen Wasserquelle oder Wasserdampfquelle; und/oder
iv) den pH-Wert eines Mediums zu bestimmen, in dem sich das mindestens eine Enzym (5) befindet, insbesondere über ein pH-Meter der Dosiereinheit (3) befindet; und/oder
v) eine Konzentration des mindestens einen Enzym (5) in einem Medium zu bestimmen, in dem sich das mindestens eine Enzym (5) befindet, insbesondere über ein UV-Spektrometer, das mit der Dosiereinheit (3) verbunden ist; und/oder
vi) ein Volumen einer Enzymlösung in der Dosiereinheit (3) zu bestimmen, insbesondere über einen Füllstandssensor, der von der Dosiereinheit (3) enthalten ist; und/oder
vii) eine Einwirkzeit des mindestens einen Enzyms (5) auf ein in der Kammer (2) befindliches biologisches Material (6) zu bestimmen.

6. 3D-Drucker (1) gemäß einem der vorhergehenden Ansprüche, **dadurch gekennzeichnet, dass** er eine Einstrahleinheit (9) enthält, die dazu geeignet ist, elektromagnetische Strahlung in die Kammer (2) zu strahlen, wobei die Einstrahleinheit (9) bevorzugt dazu geeignet ist,
a) Infrarotlicht in die Kammer (2) zu strahlen, wobei die Einstrahleinheit (9) besonders bevorzugt ausgewählt ist aus der Gruppe bestehend aus IR-Lampe, IR-Laser und Kombinationen hiervon; und/oder
b) UV-Licht in die Kammer (2) zu strahlen, wobei die Einstrahleinheit (9) besonders bevorzugt ausgewählt ist aus der Gruppe bestehend aus UV-Lampe, UV-Laser und Kombinationen hiervon;
wobei die Steuerungseinheit (4) insbesondere dazu konfiguriert ist, eine bestimmte Zeit nach dem Inkontaktbringen des mindestens einen Enzyms (5) mit dem in der Kammer (2) befindlichen biologischen Material mit der Einstrahleinheit (9) zumindest bereichsweise auf das biologische Material zu strahlen, sodass das mindestens eine Enzym (5) aktiviert oder deaktiviert wird.

7. Verfahren zum 3D-Druck eines biologischen Materials, umfassend die Schritte
a) Bereitstellen eines biologischen Materials in einer Kammer (2) eines 3D-Druckers (1), die zur Aufnahme von biologischem Material geeignet ist;
b) Bereitstellen einer Dosiereinheit (3), die zum Inkontaktbringen von mindestens einem Enzym (5) mit einem in der Kammer (2) befindlichen Material geeignet ist; und
c) Bereitstellung einer Steuerungseinheit (4) in dem 3D-Drucker (1);
**dadurch gekennzeichnet, dass** die Dosiereinheit (3) mit mindestens einem Enzym (5) ausgestattet wird, das dazu geeignet ist, eine Reaktion zu katalysieren, die dazu geeignet ist, eine kovalente Verbindung von biologischem Material zu bewirken, und die Steuerungseinheit (4) veranlasst, dass das mindestens eine Enzym (5) mit dem in der Kammer (2) befindlichen biologischen Material in Kontakt gebracht wird.

8. Verfahren gemäß Anspruch 7, **dadurch gekennzeichnet, dass** die Kammer (2)
i) einen Temperatursensor zur Messung einer Temperatur in der Kammer (2) enthält; und/oder
ii) temperiert wird, bevorzugt auf eine Temperatur im Bereich von 30 bis 60°C, besonders bevorzugt auf eine Temperatur im Bereich von 30 bis 50°C temperiert wird, insbesondere über eine Heizung (7), welche die Kammer (2) enthält; und/oder
iii) einen Drucksensor zur Messung eines Drucks in der Kammer (2) enthält; und/oder
iv) unter Druck gesetzt wird, bevorzugt auf einen Druck im Bereich von 0,1 bis 20 bar, bevorzugt 0,5 bis 15 bar, besonders bevorzugt 1 bis 10 bar, insbesondere über eine Druckluftquelle, mit der die Kammer (2) verbunden ist; und/oder
v) einen Luftfeuchtigkeitssensor zur Messung einer Luftfeuchtigkeit in der Kammer (2) enthält; und/oder
vi) einen Materialfeuchtigkeitssensor zur Messung einer Feuchtigkeit des Materials in der Kammer (2) enthält, bevorzugt einen auf einer Stromwiderstandsmessung basierenden Materialfeuchtigkeitssensor; und/oder
vii) auf eine bestimmte Feuchtigkeit eingestellt wird, insbesondere über eine Wasserquelle oder Wasserdampfquelle, die mit der die Kammer (2) verbunden ist.

9. Verfahren gemäß einem der Ansprüche 7 oder 8, **dadurch gekennzeichnet, dass** die Dosiereinheit (3)
i) in x-Richtung, y-Richtung und in z-Richtung bewegt wird, bevorzugt über eine kartesische Kinematik, besonders bevorzugt in Richtung eines Bodens (10) der Kammer (2); und/oder
ii) mit einer elektrischen Spannungsquelle zur Abgabe von elektrischer Spannung an die Dosiereinheit (3) verbunden wird und/oder mit einer Ultraschallquelle verbunden wird, wobei die Spannungsquelle und/oder Ultraschallquelle bevorzugt dazu verwendet wird, eine Freisetzung des mindestens einen Enzyms (5) in biologischen Zellen zu bewirken, bevorzugt über eine Lyse der biologischen Zellen; und/oder
iii) fluidisch mit einem Reservoir verbunden wird, das dazu geeignet ist, eine Substanz in die Dosiereinheit (3) zu geben, wobei die Substanz bevorzugt dazu geeignet ist, eine Produktion und/oder Freisetzung des mindestens einen Enzyms (5) in biologischen Zellen zu bewirken, bevorzugt über einen Transport der Substanz aus den biologischen Zellen und/oder einer Lyse der biologischen Zellen; und/oder
iv) in Richtung der Kammer (2) mit einem Sprühkopf ausgestattet wird; und/oder
v) mit einem Temperatursensor (12) zur Messung einer Temperatur in der Dosiereinheit (3) ausgestattet wird; und/oder
vi) temperierbar ist oder temperiert wird, bevorzugt auf eine Temperatur im Bereich von 1 bis 20°C, bevorzugt von 2 bis 10°C, besonders bevorzugt von 3 bis 5°C, temperierbar ist oder temperiert wird, wobei die Dosiereinheit (3) insbesondere eine Kühlung enthält; und/oder
vii) mit einem Drucksensor zur Messung eines Drucks in der Dosiereinheit (3) ausgestattet wird; und/oder
viii) unter Druck setzbar ist oder gesetzt wird, bevorzugt auf einen Druck im Bereich von 0,1 bis 20 bar, bevorzugt 0,5 bis 15 bar, besonders bevorzugt 1 bis 10 bar, insbesondere über Druckluft aus einer Druckluftquelle, mit der die Dosiereinheit (3) verbunden ist oder wird; und/oder
ix) mit einem Luftfeuchtigkeitssensor zur Messung einer Luftfeuchtigkeit in der Dosiereinheit (3) ausgestattet wird; und/oder
x) auf eine bestimmte Feuchtigkeit einstellbar ist oder eingestellt wird, wobei die Dosiereinheit (3) insbesondere über Wasser und/oder Wasserdampf aus einer Wasserquelle und/oder Wasserdampfquelle, mit der die Dosiereinheit (3) verbunden ist oder wird; und/oder
xi) mit einem pH-Meter ausgestattet wird; und/oder
xii) mit einem UV-Vis-Spektrometer verbunden wird und eine Messung einer UV-Vis-Absorption eines im Innenraum der Dosiereinheit (3) befindlichen Mediums durchgeführt wird, wobei bevorzugt eine Absorption bei einer Wellenlänge im Bereich von 240 nm bis 750 nm, bevorzugt 280 bis 700, gemessen wird; und/oder
xiii) mit einem Füllstandssensor ausgestattet wird, der zur Messung eines Volumens einer Enzymlösung in der Dosiereinheit (3) geeignet ist; und/oder
xiv) mit Molekülen ausgestattet wird, welche die kovalenten Verbindungen des biologischen Materials begünstigen, bevorzugt ein Molekül ausgewählt aus der Gruppe bestehend aus Enzymsubstraten, Enzym-Cofaktoren, chemisch aktivierten Molekülen und Mischungen hiervon, wobei die Enzymsubstrate insbesondere ausgewählt sind aus Kohlenhydraten, Proteinen, Peptiden, Nukleinsäuren, Lipiden, Phenolen, Phenylpropanoiden und Mischungen hiervon, optional Monomere, Oligomere und/oder Polymere dieser Moleküle, der Enzym-Cofaktor insbesondere Lysyl-Tyrosyl-Chinon ist und/oder die Enzymsubstrate insbesondere chemisch aktivierte Moleküle sind; und/oder
xv) keine Naturfasern enthält, bevorzugt kein biologisches Material (6) enthält, das kovalent durch eine von dem mindestens einen Enzym (5) katalysiere Reaktion verbunden wird;
xvi) kein hygroskopisches Mineral enthält, bevorzugt kein hygroskopisches Material enthält; und/oder
xvii) einen öffenbaren Verschluss in Richtung Kammer (2) aufweist, wobei bevorzugt die Steuerungseinheit (4) veranlasst, den öffenbaren Verschluss zu öffnen, besonders bevorzugt basierend auf einem Befehl eines Benutzers.

10. Verfahren gemäß einem der Ansprüche 7 bis 9, **dadurch gekennzeichnet, dass** das mindestens eine Enzym (5)
i) in gelöster oder fester Form vorliegt, bevorzugt gelöst in einer wässrigen Pufferlösung oder einem wässrigen Fermentationsmedium oder in Form eines festen Pulvers; und/oder
ii) von festen Filamenten enthalten ist und/oder in festen Filamenten immobilisiert ist; und/oder
iii) in biologischen Zellen enthalten ist und/oder in der Zellmembran von biologischen Zellen enthalten ist, wobei die biologischen Zellen bevorzugt ausgewählt sind aus der Gruppe bestehend aus prokaryotischen Zellen, eukaryotischen Zelle und Mischungen hiervon, besonders bevorzugt ausgewählt aus der Gruppe bestehend aus Bakterienzellen, Archaeenzellen, pilzlichen, pflanzlichen oder tierischen Zellen und Mischungen hiervon; und/oder
iv) in einem Innenraum der Dosiereinheit (3) bereitgestellt wird; und/oder
v) in der Kammer (2) bereitgestellt wird; und/oder
vi) an einer der Kammer (2) zugewandten äußeren Wand der Dosiereinheit (3) immobilisiert bereitgestellt wird; und/oder
vii) ausgewählt ist aus der Gruppe bestehend aus Laccasen, Tyrosinase, Peroxidasen, Phenoloxidasen, Aminosäureoxidasen, Transglutaminasen, und Mischungen hiervon.

11. Verfahren gemäß einem der Ansprüche 7 bis 10, **dadurch gekennzeichnet, dass** über die Steuerungseinheit (4)
i) eine Temperatur der Kammer (2) und/oder Dosiereinheit (3) regelt, bevorzugt auf eine Temperatur im Bereich von 30 bis 60°C, besonders bevorzugt von 30 bis 50°C, für die Kammer (2) und/oder auf eine Temperatur im Bereich von 1 bis 20°C, bevorzugt von 2 bis 10°C, besonders bevorzugt von 3 bis 5°C, für die Dosiereinheit (3), insbesondere über Steuerung einer Heizung (7) und/oder Kühlung (8) der Kammer (2) und/oder Dosiereinheit (3);
ii) ein Druck in der Kammer (2) und/oder Dosiereinheit (3) geregelt wird, bevorzugt auf einen Druck im Bereich von 0,1 bis 20 bar, bevorzugt 0,5 bis 15 bar, besonders bevorzugt 1 bis 10 bar, insbesondere über Steuerung einer mit der Kammer (2) und/oder Dosiereinheit (3) verbundenen Druckluftquelle; und/oder
iii) eine Feuchtigkeit in der Kammer (2) und/oder Dosiereinheit (3) geregelt wird, insbesondere über eine mit der Kammer (2) und/oder Dosiereinheit (3) verbundenen Wasserquelle oder Wasserdampfquelle; und/oder
iv) der pH-Wert eines Medium bestimmt wird, in dem sich das mindestens eine Enzym (5) befindet, insbesondere über ein pH-Meter der Dosiereinheit (3); und/oder
v) eine Konzentration des mindestens einen Enzym (5) in einem Medium bestimmt wird, in dem sich das mindestens eine Enzym (5) befindet, insbesondere über ein UV-Spektrometer, das mit der Dosiereinheit (3) verbunden ist; und/oder
vi) ein Volumen einer Enzymlösung in der Dosiereinheit (3) bestimmt wird, insbesondere über einen Füllstandssensor, der von der Dosiereinheit (3) enthalten ist; und/oder
vii) eine Einwirkzeit des mindestens einen Enzyms (5) auf ein in der Kammer (2) befindliches biologisches Material (6) bestimmt wird.

12. Verfahren gemäß einem der Ansprüche 7 bis 11, **dadurch gekennzeichnet, dass** der 3D-Drucker (1) mit einer Einstrahleinheit (9) ausgestattet wird, die dazu geeignet ist, elektromagnetische Strahlung in die Kammer (2)zu strahlen, wobei die Einstrahleinheit (9) bevorzugt dazu geeignet ist,
a) Infrarotlicht in die Kammer (2) zu strahlen, wobei die Einstrahleinheit (9) besonders bevorzugt ausgewählt ist aus der Gruppe bestehend aus IR-Lampe, IR-Laser und Kombinationen hiervon; und/oder
b) UV-Licht in die Kammer (2) zu strahlen, wobei die Einstrahleinheit (9) besonders bevorzugt ausgewählt ist aus der Gruppe bestehend aus UV-Lampe, UV-Laser und Kombinationen hiervon;
wobei eine bestimmte Zeit nach dem Inkontaktbringen des mindestens einen Enzyms (5) mit dem in der Kammer (2) befindlichen biologischen Material mit der Einstrahleinheit (9) zumindest bereichsweise auf das biologische Material gestrahlt wird, sodass das mindestens eine Enzym (5) aktiviert oder deaktiviert wird.

13. Extruder, Spritzgießer oder Formpresse, enthaltend
a) eine Kammer zur Aufnahme von einem Material, optional enthaltend ein biologisches Material;
b) eine Dosiereinheit zum Inkontaktbringen von mindestens einem Enzym mit einem in der Kammer befindlichen Material; und
c) eine Steuerungseinheit;
**dadurch gekennzeichnet, dass** die Dosiereinheit mindestens ein Enzym enthält, das dazu geeignet ist, eine Reaktion zu katalysieren, die dazu geeignet ist, eine kovalente Verbindung von biologischem Material zu bewirken, und die Steuerungseinheit dazu konfiguriert ist, das Inkontaktbringen des mindestens einen Enzyms mit einem in der Kammer befindlichen biologischen Material auf eine Art und Weise zu veranlassen, dass die Gesamtmenge von Enzym in einem Bereich von 0,001 Gew.-% bis <0,01 Gew.-%, bezogen auf die Gesamtmenge aus biologischem Material und Enzym in der Kammer, liegt.

14. Verfahren zur Extrusion, zum Formpressen oder zum Spritzguss eines biologischen Materials, umfassend die Schritte
a) Bereitstellen eines biologischen Materials in einer Kammer eines Extruders, einer Formpresse oder eines Spritzgießers, wobei die Kammer zur Aufnahme von biologischem Material geeignet ist;
b) Bereitstellen einer Dosiereinheit, die zum Inkontaktbringen von mindestens einem Enzym mit dem in der Kammer befindlichen biologischen Material geeignet ist; und
c) Bereitstellung einer Steuerungseinheit;
**dadurch gekennzeichnet, dass** die Dosiereinheit mit mindestens einem Enzym ausgestattet wird, das dazu geeignet ist, eine Reaktion zu katalysieren, die dazu geeignet ist, eine kovalente Verbindung von biologischem Material zu bewirken, und die Steuerungseinheit veranlasst, dass das mindestens eine Enzym mit dem in der Kammer befindlichen biologischen Material auf eine Art und Weise in Kontakt gebracht wird, dass die Gesamtmenge von Enzym in einem Bereich von 0,001 Gew.-% bis <0,01 Gew.-%, bezogen auf die Gesamtmenge aus biologischem Material und Enzym in der Kammer, liegt.

15. Biologisches Material, das über ein Verfahren gemäß einem der Ansprüche 7 bis 12 bei dem mehrere Schichten von biologischem Material über Enzymeinwirkung miteinander verbunden wurden, hergestellt wurde,
**dadurch gekennzeichnet, dass** das biologische Material einen Schichtaufbau aufweist, wobei sich zwischen den Schichten des verbundenen biologischen Materials das Enzym befindet, das dazu geeignet ist, eine Reaktion zu katalysieren, die dazu geeignet ist, eine kovalente Verbindung von biologischem Material zu bewirken,
wobei die Gesamtmenge des Enzyms, das dazu geeignet ist, eine Reaktion zu katalysieren, die dazu geeignet ist, eine kovalente Verbindung von biologischem Material zu bewirken, in einem Bereich von 0,001 Gew.-% bis <0,01 Gew.-%, bezogen auf die Gesamtmenge von Enzym und biologischem Material, liegt.

## Claims

1. A 3D printer (1), comprising:
a) a chamber (2), optionally containing a biological material (6);
b) a dosing unit (3) for bringing at least one enzyme (5) in contact with a material present in the chamber (2); and
c) a control unit (4),
**characterized in that** the dosing unit (3) contains at least one enzyme (5) suitable for catalyzing a reaction suitable for effectuating a covalent bonding of biological material, and the control unit (4) is configured to prompt the process of bringing the at least one enzyme (5) in contact with a biological material present in the chamber (2).

2. The 3D printer (1) according to claim 1, **characterized in that** the chamber (2)
i) comprises a temperature sensor (11) for measuring a temperature in the chamber (2); and/or
ii) can be controlled in terms of the temperature, preferably to a temperature in the range of 30 to 60°C, particularly preferably to a temperature in the range of 30 to 50°C, the chamber (2) in particular comprising a heater (7); and/or
iii) comprises a pressure sensor for measuring a pressure in the chamber (2); and/or
iv) can be pressurized, preferably to a pressure in the range of 0.1 to 20 bar, preferably 0.5 to 15 bar, particularly preferably 1 to 10 bar, the chamber (2) in particular being connected to a compressed air source; and/or
v) comprises an air moisture sensor for measuring an air moisture in the chamber (2); and/or
vi) comprises a material moisture sensor for measuring a moisture of the material in the chamber (2), preferably a material moisture sensor based on an electrical resistance measurement; and/or
vii) is settable to a certain moisture level, the chamber (2) in particular being connected to a water source or water vapor source; and/or
viii) is suitable of being dehumidified, the chamber (2) in particular being connected to a condenser and/or a desiccant.

3. The 3D printer (1) according to any one of the preceding claims, **characterized in that** the dosing unit (3)
i) is designed so as to be movable in the x direction, y direction and z direction, preferably by way of a Cartesian kinematic system, and particularly preferably is designed so as to be movable in the direction of a bottom (10) of the chamber (2); and/or
ii) is connected to an electrical voltage source for delivering voltage to the dosing unit (3) and/or is connected to an ultrasound source, the voltage source and/or ultrasound source preferably being suitable for effectuating a release of the at least one enzyme (5) in biological cells, preferably by way of lysis of the biological cells; and/or
iii) is fluidically connected to a reservoir which is suitable for adding a substance to the dosing unit (3), the substance preferably being suitable for effectuating a production and/or release of the at least one enzyme (5) in biological cells, preferably by way of a transport of the substance out of the biological cells and/or lysis of the biological cells; and/or
iv) comprises a spray head in the direction of the chamber (2);
and/or
v) comprises a temperature sensor for measuring a temperature in the dosing unit (3); and/or
vi) is controllable in terms of the temperature, preferably to a temperature of 1 to 20°C, preferably in the range of 2 to 10°C, particularly preferably in the range of 3°C to 5°C, the dosing unit (3) in particular comprising a cooling unit; and/or
vii) comprises a pressure sensor for measuring a pressure in the dosing unit (3); and/or
viii) is suitable of being pressurized, preferably to a pressure in the range of 0.1 to 20 bar, preferably 0.5 to 15 bar, particularly preferably 1 to 10 bar, the dosing unit (3) in particular being connected to a compressed air source; and/or
ix) comprises an air moisture sensor for measuring an air moisture in the dosing unit (3); and/or
x) is settable to a certain moisture level, the dosing unit (3) in particular being connected to a water source or water vapor source; and/or
xi) comprises a pH meter; and/or
xii) is connected to a UV/Vis spectrometer, which is suitable for measuring light absorption of a medium present in the interior of the dosing unit (3), the UV/Vis spectrometer preferably being configured to measure an absorption at a wavelength in the range of 240 to 750 nm, preferably 280 nm to 700 nm; and/or
xiii) comprises a fill level sensor, which is suitable for measuring a volume of an enzyme solution in the dosing unit (3); and/or
xiv) contains molecules which facilitate the covalent bonding of the biological material, preferably a molecule selected from the group consisting of enzyme substrates, enzyme cofactors, chemically activated molecules and mixtures thereof, the enzyme substrates in particular being selected from the group consisting of carbohydrates, proteins, peptides, nucleic acids, lipids, phenols, phenylpropanoids and mixtures thereof, optionally monomers, oligomers and/or polymers of these molecules, and/or the enzyme cofactor in particular being lysyl tyrosylquinone and/or the enzyme substrates in particular being chemically activated molecules; and/or
xv) does not contain natural fibers, preferably does not contain biological material (6) that will covalently bond by a reaction catalyzed by the at least one enzyme (5); and/or
xvi) does not contain hygroscopic mineral, and preferably does not contain hygroscopic material; and/or
xvii) comprises a openable closure in the direction of the chamber (2), the control unit (4) preferably being configured to prompt the openable closure to be opened, particularly preferably based on a command of a user.

4. The 3D printer (1) according to any one of the preceding claims, **characterized in that** the at least one enzyme (5)
i) is present in dissolved or solid form, preferably dissolved in an aqueous buffer solution or an aqueous fermentation medium or in the form of a solid powder; and/or
ii) is contained in solid filaments and/or is immobilized in solid filaments; and/or
iii) is contained in biological cells and/or is contained in the cell membrane of biological cells, the biological cells preferably being selected from the group consisting of prokaryotic cells, eukaryotic cells and mixtures thereof, particularly preferably selected from the group consisting of bacterial cells, archaean cells, fungal, plant and animal cells and mixtures thereof; and/or
iv) is present in an interior of the dosing unit (3); and/or
v) is present in the chamber (2); and/or
vi) is immobilized on an outer wall of the dosing unit (3) which faces the chamber (2); and/or
vii) is selected from the group consisting of laccases, tyrosinase, peroxidases, phenoloxidases, amino acid oxidases, transglutaminases and mixtures thereof.

5. The 3D printer (1) according to any one of the preceding claims, **characterized in that** the control unit (4) is configured
i) to regulate a temperature of the chamber (2) and/or dosing unit (3), preferably to a temperature in the range of 30 to 60°C, particularly preferably of 30 to 50°C for the chamber (2) and/or to a temperature in the range of 1 to 20°C, preferably of 2 to 10°C, particularly preferably of 3 to 5°C for the dosing unit (3), in particular by controlling a heater (7) and/or cooling unit (8) of the chamber (2) and/or dosing unit (3); and/or
ii) to regulate a pressure in the chamber (2) and/or dosing unit (3), preferably to a pressure in the range of 0.1 to 20 bar, preferably 0.5 to 15 bar, particularly preferably 1 to 10 bar, in particular by controlling a compressed air source connected to the chamber (2) and/or dosing unit (3); and/or
iii) to regulate a moisture level in the chamber (2) and/or dosing unit (3), in particular via a water source or water vapor source connected to the chamber (2) and/or dosing unit (3); and/or
iv) to determine the pH value of a medium in which the at least one enzyme (5) is present, in particular by way of a pH meter of the dosing unit (3); and/or
v) to determine a concentration of the at least one enzyme (5) in a medium in which the at least one enzyme (5) is present, in particular by way of a UV spectrometer connected to the dosing unit (3); and/or
vi) to determine a volume of an enzyme solution in the dosing unit (3), in particular by way of a fill level sensor contained in the dosing unit (3); and/or
vii) to determine an exposure time of a biological material (6) present in the chamber (2) to the at least one enzyme (5).

6. The 3D printer (1) according to any one of the preceding claims, **characterized in that** it comprises an irradiation unit (9), which is suitable for irradiating electromagnetic radiation into the chamber (2), the irradiation unit (9) preferably being suitable for
a) irradiating infrared light into the chamber (2), the irradiation unit (9) particularly preferably being selected from the group consisting of IR lamp, IR laser and combinations thereof; and/or
b) irradiating UV light into the chamber (2), the irradiation unit (9) particularly preferably being selected from the group consisting of UV lamp, UV laser and combinations thereof,
the control unit (4) being in particular configured to have the biological material irradiated at least regionally by way of the irradiation unit (9) for a certain time after the at least one enzyme (5) has been brought in contact with the biological material present in the chamber (2) so that the at least one enzyme (5) is activated or deactivated.

7. A method for 3D printing a biological material is, comprising the following steps:
a) providing a biological material in a chamber (2) of a 3D printer (1), the chamber being suitable for receiving biological material;
b) providing a dosing unit (3) suitable for bringing at least one enzyme (5) in contact with a material present in the chamber (2); and
c) providing a control unit (4) in the 3D printer (1),
**characterized in that** the dosing unit (3) is furnished with at least one enzyme (5) suitable for catalyzing a reaction suitable for effectuating a covalent bonding of biological material, and the control unit (4) prompts the process of bringing the at least one enzyme (5) in contact with the biological material present in the chamber (2).

8. The method according to claim 7, **characterized in that** the chamber (2)
i) comprises a temperature sensor for measuring a temperature in the chamber (2); and/or
ii) is controlled in terms of the temperature, preferably to a temperature in the range of 30 to 60°C, particularly preferably is controlled to a temperature in the range of 30 to 50°C, in particular by way of a heater (7) contained in the chamber (2); and/or
iii) comprises a pressure sensor for measuring a pressure in the chamber (2); and/or
iv) is pressurized, preferably to a pressure in the range of 0.1 to 20 bar, preferably 0.5 to 15 bar, particularly preferably 1 to 10 bar, in particular by way of a compressed air source connected to the chamber (2); and/or
v) comprises an air moisture sensor for measuring an air moisture in the chamber (2); and/or
vi) comprises a material moisture sensor for measuring a moisture of the material in the chamber (2), preferably material moisture sensor based on an electrical resistance measurement; and/or
vii) is set to a particular moisture level, in particular by way of a water source or water vapor source to which the chamber (2) is connected.

9. The method according to either claim 7 or 8, **characterized in that** the dosing unit (3)
i) is moved in the x direction, y direction and z direction, preferably by way of a Cartesian kinematic system, and particularly preferably in the direction of a bottom (10) of the chamber (2); and/or
ii) is connected to an electrical voltage source for delivering voltage to the dosing unit (3) and/or is connected to an ultrasound source, the voltage source and/or ultrasound source preferably being used to effectuate a release of the at least one enzyme (5) in biological cells, preferably by way of lysis of the biological cells; and/or
iii) is fluidically connected to a reservoir which is suitable for adding a substance to the dosing unit (3), the substance preferably being suitable for effectuating a production and/or release of the at least one enzyme (5) in biological cells, preferably by way of a transport of the substance out of the biological cells and/or lysis of the biological cells; and/or
iv) is equipped with a spray head in the direction of the chamber (2); and/or
v) is equipped with a temperature sensor (12) for measuring a temperature in the dosing unit (3); and/or
vi) is controllable or is controlled in terms of the temperature, preferably is controllable or is controlled to a temperature in the range of 1 to 20°C, preferably of 2 to 10°C, particularly preferably of 3 to 5°C, the dosing unit (3) in particular comprising a cooling unit; and/or
vii) is equipped with a pressure sensor for measuring a pressure in the dosing unit (3); and/or
viii) is suitable to be pressurized or is pressurized, preferably to a pressure in the range of 0.1 to 20 bar, preferably 0.5 to 15 bar, particularly preferably 1 to 10 bar, in particular by way of compressed air from a compressed air source to which the dosing unit (3) is connected or is being connected; and/or
ix) is equipped with an air moisture sensor for measuring an air moisture in the dosing unit (3); and/or
x) is settable or be set to a certain moisture level, the dosing unit (3) in particular by way of water and/or water vapor from a water source and/or water vapor source to which the dosing unit (3) is being connected; and/or
xi) is equipped with a pH meter; and/or
xii) is connected to a UV/Vis spectrometer and a measurement of a UV/Vis absorption of a medium present in the interior of the dosing unit (3) is carried out, an absorption being measured at a wavelength in the range of 240 nm to 750 nm, preferably 280 to 700 nm; and/or
xiii) is equipped with a fill level sensor, which is suitable for measuring a volume of an enzyme solution in the dosing unit (3); and/or
xiv) is furnished with molecules which facilitate the covalent bonding of the biological material, preferably a molecule selected from the group consisting of enzyme substrates, enzyme cofactors, chemically activated molecules and mixtures thereof, the enzyme substrates in particular being selected from carbohydrates, proteins, peptides, nucleic acids, lipids, phenols, phenylpropanoids and mixtures thereof, optionally monomers, oligomers and/or polymers of these molecules, the enzyme cofactor in particular being lysyl tyrosylquinone and/or the enzyme substrates in particular being chemically activated molecules; and/or
xv) does not contain natural fibers, preferably does not contain biological material (6) that will covalently bond by a reaction catalyzed by the at least one enzyme (5);
xvi) does not contain hygroscopic mineral, and preferably does not contain hygroscopic material; and/or
xvii) comprises a openable closure in the direction of the chamber (2), the control unit (4) prompting the openable closure to be opened, particularly preferably based on a command of a user.

10. The method according to any one of claims 7 to 9, **characterized in that** the at least one enzyme (5)
i) is present in dissolved or solid form, preferably dissolved in an aqueous buffer solution or an aqueous fermentation medium or in the form of a solid powder; and/or
ii) is contained in solid filaments and/or is immobilized in solid filaments; and/or
iii) is contained in biological cells and/or is contained in the cell membrane of biological cells, the biological cells preferably being selected from the group consisting of prokaryotic cells, eukaryotic cells and mixtures thereof, particularly preferably selected from the group consisting of bacterial cells, archaean cells, fungal, plant or animal cells and mixtures thereof; and/or
iv) is provided in an interior of the dosing unit (3); and/or
v) is provided in the chamber (2); and/or
vi) is provided immobilized on an outer wall of the dosing unit (3) which faces the chamber (2); and/or
vii) is selected from the group consisting of laccases, tyrosinase, peroxidases, phenoloxidases, amino acid oxidases, transglutaminases and mixtures thereof.

11. The method according to any one of claims 7 to 10, **characterized in that** the control unit (4)
i) is used to regulate a temperature of the chamber (2) and/or dosing unit (3), preferably to a temperature in the range of 30 to 60°C, particularly preferably of 30 to 50°C for the chamber (2) and/or to a temperature in the range of 1 to 20°C, preferably of 2 to 10°C, particularly preferably of 3 to 5°C, for the dosing unit (3), in particular by controlling a heater (7) and/or cooling unit (8) of the chamber (2) and/or dosing unit (3);
ii) is used to regulate a pressure in the chamber (2) and/or dosing unit (3), preferably to a pressure in the range of 0.1 to 20 bar, preferably 0.5 to 15 bar, particularly preferably 1 to 10 bar, in particular by controlling a compressed air source connected to the chamber (2) and/or dosing unit (3); and/or
iii) is used to regulate a moisture level in the chamber (2) and/or dosing unit (3), in particular via a water source or water vapor source connected to the chamber (2) and/or dosing unit (3); and/or
iv) is used to determine the pH value of a medium in which the at least one enzyme (5) is present, in particular by way of a pH meter of the dosing unit (3); and/or
v) is used to determine a concentration of the at least one enzyme (5) in a medium in which the at least one enzyme (5) is present, in particular by way of a UV spectrometer connected to the dosing unit (3); and/or
vi) is used to determine a volume of an enzyme solution in the dosing unit (3), in particular by way of a fill level sensor contained in the dosing unit (3); and/or
vii) is used to determine an exposure time of a biological material (6) present in the chamber (2) to the at least one enzyme (5).

12. The method according to any one of claims 7 to 11, **characterized in that** the 3D printer (1) is equipped with an irradiation unit (9), which is suitable for irradiating electromagnetic radiation into the chamber (2), the irradiation unit (9) preferably being suitable for
a) irradiating infrared light into the chamber (2), the irradiation unit (9) particularly preferably being selected from the group consisting of IR lamp, IR laser and combinations thereof; and/or
b) irradiating UV light into the chamber (2), the irradiation unit (9) particularly preferably being selected from the group consisting of UV lamp, UV laser and combinations thereof,
the biological material being irradiated at least regionally by way of the irradiation unit (9) for a certain time after the at least one enzyme (5) has been brought in contact with the biological material present in the chamber (2) so that at least one enzyme (5) is activated or deactivated.

13. An extruder, an injection molding machine or a compression molding machine, comprising:
a) a chamber for receiving material, optionally containing a biological material;
b) a dosing unit for bringing at least one enzyme in contact with a material present in the chamber; and
c) a control unit,
**characterized in that** the dosing unit contains at least one enzyme suitable for catalyzing a reaction suitable for effectuating a covalent bonding of biological material, and the control unit is configured to prompt the process of bringing the at least one enzyme in contact with a biological material present in the chamber in such a manner that the total amount of enzyme is in a range of 0.001 wt.% to <0.01 wt.%, based on the total amount of biological material and enzyme in the chamber.

14. A method for extruding, for compression molding or for injection molding of a biological material, comprising the following steps:
a) providing a biological material in a chamber of an extruder, of a compression molding machine or an injection molding machine, the chamber being suitable for receiving biological material;
b) providing a dosing unit suitable for bringing at least one enzyme in contact with the biological material present in the chamber; and
c) providing a control unit,
**characterized in that** the dosing unit is furnished with at least one enzyme suitable for catalyzing a reaction suitable for effectuating a covalent bonding of biological material, and the control unit prompts the process of bringing the at least one enzyme in contact with the biological material present in the chamber in such a manner that the total amount of enzyme is in a range of 0.001 wt.% to <0.01 wt.%, based on the total amount of biological material and enzyme in the chamber.

15. A biological material, produced by way of a method according to any one of claims 7 to 12 in which a plurality of layers of biological material were bonded to one another by enzyme action,
**characterized in that** the biological material has a layer composition, the enzyme which is suitable for catalyzing a reaction suitable for effectuating a covalent bonding of biological material being present between the layers of the bonded biological material,
the total amount of the enzyme which is suitable for catalyzing a reaction suitable for effectuating a covalent bonding of biological material being in a range of 0.001 wt.% to <0.01 wt.%, based on the total amount of enzyme and biological material.

## Revendications

1. Imprimante 3D (1), contenant
a) une chambre (2), contenant facultativement un matériau biologique (6) ;
b) une unité de dosage (3) pour mettre en contact au moins une enzyme (5) avec un matériau situé dans la chambre (2) ; et
c) une unité de commande (4) ;
**caractérisé en ce que** l'unité de dosage (3) contient au moins une enzyme (5) qui est adaptée pour catalyser une réaction qui est adaptée pour effectuer une liaison covalente de matériau biologique, et l'unité de commande (4) est configurée pour provoquer la mise en contact de la au moins une enzyme (5) avec un matériau biologique situé dans la chambre (2).

2. Imprimante 3D (1) selon la revendication 1, **caractérisée en ce que** la chambre (2)
i) contient un capteur de température (11) pour mesurer une température dans la chambre (2) ; et/ou
ii) peut être tempérée, de préférence à une température comprise entre 30 et 60°C, de manière particulièrement préférée jusqu'à une température comprise entre 30 et 50°C, la chambre (2) contenant notamment un élément chauffant (7) ; et/ou
iii) contient un capteur de pression pour mesurer une pression dans la chambre (2) ; et/ou
iv) peut être mise sous pression, de préférence à une pression comprise entre 0,1 et 20 bars, de préférence entre 0,5 et 15 bars, de manière particulièrement préférée entre 1 et 10 bars, la chambre (2) étant reliée notamment à une source d'air comprimé ; et/ou
v) contient un capteur d'humidité pour mesurer une humidité dans la chambre (2) ; et/ou
vi) contient un capteur d'humidité de matériau pour mesurer une humidité du matériau dans la chambre (2), de préférence un capteur d'humidité de matériau basé sur une mesure de résistance de courant ; et/ou
vii) peut être ajustée à une humidité spécifique, la chambre (2) étant reliée notamment à une source d'eau ou à une source de vapeur d'eau ; et/ou
viii) peut être déshumidifiée, la chambre (2) étant reliée notamment à un condenseur et/ou un dessiccatif.

3. Imprimante 3D (1) selon l'une des revendications précédentes, **caractérisée en ce que** l'unité de dosage (3)
i) est conçue pour être mobile dans une direction x, dans une direction y et dans une direction z, de préférence est conçue pour être mobile via une cinématique cartésienne, de manière particulièrement préférée en direction d'un sol (10), de la chambre (2) ; et/ou
ii) est connectée à une source de tension électrique pour fournir une tension électrique à l'unité de dosage (3) et/ou est connectée à une source d'ultrasons, la source de tension et/ou la source d'ultrasons étant de préférence adaptées pour libérer la au moins une enzyme (5) dans des cellules biologiques, de préférence par lyse des cellules biologiques ; et/ou
iii) est reliée de manière fluidique à un réservoir qui est adapté pour ajouter une substance dans l'unité de dosage (3), la substance étant de préférence appropriée pour effectuer une production et/ou libération de la ou des enzymes (5) dans des cellules biologiques, de préférence via un transport de la substance hors des cellules biologiques et/ou une lyse des cellules biologiques ; et/ou
iv) présente une tête de pulvérisation en direction de la chambre (2) ; et/ou
v) contient un capteur de température pour mesurer une température dans l'unité de dosage (3) ; et/ou
vi) peut être tempérée, de préférence à une température comprise entre 1 et 20°C, de préférence comprise entre 2 et 10°C, de manière particulièrement préférée comprise entre 3°C et 5°C, l'unité de dosage (3) contenant en particulier un système de refroidissement ; et/ou
vii) contient un capteur de pression pour mesurer une pression dans l'unité de dosage (3) ; et/ou
viii) peut être mise sous pression, de préférence à une pression comprise entre 0,1 et 20 bars, de préférence entre 0,5 et 15 bars, de manière particulièrement préférée entre 1 et 10 bars, l'unité de dosage (3) étant reliée notamment à une source d'air comprimé ; et ou
ix) contient un capteur d'humidité pour mesurer une humidité dans l'unité de dosage (3) ; et/ou
x) peut être ajustée à une humidité spécifique, l'unité de dosage (3) étant reliée notamment à une source d'eau ou à une source de vapeur d'eau ; et/ou
xi) présente un pH-mètre ; et/ou
xii) est reliée à un spectromètre UV-Vis, qui est approprié pour mesurer une absorption de lumière d'un milieu situé à l'intérieur de l'unité de dosage (3), le spectromètre UV-Vis étant de préférence configuré pour mesurer une absorption à une longueur d'onde comprise entre 240 et 750 nm, de préférence comprise entre 280 nm et 700 nm ; et/ou
xiii) présente un capteur de niveau de remplissage qui est adapté pour mesurer un volume d'une solution enzymatique dans l'unité de dosage (3) ; et/ou
xiv) contient des molécules qui favorisent la liaison covalente du matériau biologique, de préférence une molécule choisie dans le groupe constitué par des substrats enzymatiques, des cofacteurs enzymatiques, des molécules activées chimiquement et des mélanges de ceux-ci, les substrats enzymatiques étant notamment choisis dans le groupe constitué par des glucides, des protéines, des peptides, des acides nucléiques, des lipides, des phénols, des phénylpropanoïdes et des mélanges de ceux-ci, facultativement des monomères, des oligomères et/ou des polymères de ces molécules et/ou le cofacteur enzymatique est notamment la lysyl-tyrosyl-quinone et/ou les substrats enzymatiques sont notamment des molécules activées chimiquement ; et/ou
xv) ne contient aucune fibre naturelle, de préférence ne contient aucun matériau biologique (6) qui est lié de manière covalente par une réaction catalysée par ladite au moins une enzyme (5) ; et/ou
xvi) ne contient aucun minéral hygroscopique, de préférence ne contient aucun matériau hygroscopique ; et/ou
xvii) présente une fermeture pouvant être ouverte en direction de la chambre (2), l'unité de commande (4) étant de préférence configurée pour provoquer une ouverture de la fermeture pouvant être ouverte, de manière particulièrement préférée sur la base d'une commande d'un utilisateur.

4. Imprimante 3D (1) selon l'une des revendications précédentes, **caractérisée en ce que** la au moins une enzyme (5)
i) est sous forme dissoute ou solide, de préférence dissoute dans une solution tampon aqueuse ou un milieu de fermentation aqueux ou sous la forme d'un poudre solide ; et/ou
ii) est contenue par des filaments solides et/ou est immobilisée dans des filaments solides ; et/ou
iii) est contenue dans des cellules biologiques et/ou est contenue dans la membrane cellulaire de cellules biologiques, les cellules biologiques étant de préférence sélectionnées dans le groupe constitué par des cellules procaryotes, des cellules eucaryotes et des mélanges de celles-ci, de manière particulièrement préférée sélectionnées dans le groupe constitué par des cellules bactériennes, des cellules archées, des cellules fongiques, végétales et animales et des mélanges de celles-ci ; et/ou
iv) est présente dans un espace intérieur de l'unité de dosage (3) ; et/ou
v) se situe dans la chambre (2) ; et/ou
vi) est immobilisée sur une paroi externe de l'unité de dosage (3) faisant face à la chambre (2) ; et/ou
vii) est choisie dans le groupe constitué des laccases, des tyrosinases, des peroxydases, des phénoloxydases, des oxydases d'acides aminés, des transglutaminases et des mélanges de celles-ci.

5. Imprimante 3D (1) selon l'une des revendications précédentes, **caractérisée en ce que** l'unité de commande (4) est configurée pour
i) réguler une température de la chambre (2) et/ou de l'unité de dosage (3), de préférence à une température comprise entre 30 et 60°C, de manière particulièrement préférée comprise entre 30 et 50°C, pour la chambre (2) et/ ou à une Température comprise entre 1 et 20°C, de préférence comprise entre 2 et 10°C, de manière particulièrement préférée comprise entre 3 et 5°C pour l'unité de dosage (3), notamment via la commande d'un élément chauffant (7) et/ ou d'un système de refroidissement (8) de la chambre (2) et/ou de l'unité de dosage (3) ; et/ou
ii) réguler une pression dans la chambre (2) et/ou dans l'unité de dosage (3), de préférence à une pression comprise entre 0,1 et 20 bars, de préférence comprise entre 0,5 et 15 bars, de manière particulièrement préférée comprise entre 1 à 10 bars, notamment via une commande d'une source d'air comprimé reliée à la chambre (2) et/ou à l'unité de dosage (3) ; et/ou
iii) réguler une humidité dans la chambre (2) et/ou l'unité de dosage (3), notamment via une source d'eau ou une source de vapeur d'eau reliée à la chambre (2) et/ou à l'unité de dosage (3) ; et/ou
iv) déterminer la valeur de pH d'un milieu dans lequel se trouve la au moins une enzyme (5), notamment via un pH-mètre de l'unité de dosage (3) ; et/ou
v) déterminer une concentration de la au moins une enzyme (5) dans un milieu dans lequel se trouve la au moins une enzyme (5), notamment via un spectromètre UV qui est relié à l'unité de dosage (3) ; et/ou
vi) déterminer un volume d'une solution enzymatique dans l'unité de dosage (3), notamment via un capteur de niveau de remplissage qui est contenu dans l'unité de dosage (3) ; et/ou
vii) déterminer un temps d'exposition de la au moins une enzyme (5) à un matériau biologique (6) situé dans la chambre (2).

6. Imprimante 3D (1) selon l'une des revendications précédentes, **caractérisée en ce qu'**elle contient une unité de rayonnement (9) qui est adaptée pour rayonner un rayonnement électromagnétique dans la chambre (2), l'unité de rayonnement (9) étant de préférence adaptée à cet effet,
a) pour rayonner une lumière infrarouge dans la chambre (2), l'unité de rayonnement (9) étant de manière particulièrement préférée choisie dans le groupe constitué d'une lampe IR, d'un laser IR et de combinaisons de ceux-ci ; et/ou
b) pour rayonner de la lumière UV dans la chambre (2), l'unité de rayonnement (9) étant choisie de manière particulièrement préférée dans le groupe constitué d'une lampe UV, d'un laser UV et de combinaisons de ceux-ci ;
dans laquelle l'unité de commande (4) est configurée en particulier, un certain temps après la mise en contact de la au moins une enzyme (5) avec le matériau biologique situé dans la chambre (2), pour rayonner sur le matériau biologique à l'aide de l'unité de rayonnement (9) au moins par endroits, de sorte que la au moins une enzyme (5) soit activée ou désactivée.

7. Procédé d'impression 3D d'un matériau biologique, comprenant les étapes consistant à
a) fournir un matériau biologique dans une chambre (2) d'une imprimante 3D (1) qui est adaptée pour contenir du matériau biologique ;
b) fournir une unité de dosage (3) qui est adaptée pour mettre en contact au moins une enzyme (5) avec un matériau situé dans la chambre (2) ; et
c) fournir une unité de commande (4) dans l'imprimante 3D (1) ;
**caractérisé en ce que** l'unité de dosage (3) est équipée d'au moins une enzyme (5) qui est adaptée pour catalyser une réaction qui est adaptée pour effectuer une liaison covalente de matériau biologique, et l'unité de commande (4) provoque la mise en contact de la au moins une enzyme (5) avec le matériau biologique situé dans la chambre (2).

8. Procédé selon la revendication 7, **caractérisé en ce que** la chambre (2)
i) contient un capteur de température pour mesurer une température dans la chambre (2) ; et/ou
ii) est tempérée, de préférence à une température comprise entre 30 et 60 °C, de manière particulièrement préférée jusqu'à une température comprise entre 30 et 50°C, notamment via un élément chauffant (7) que contient la chambre (2) ; et/ou
iii) contient un capteur de pression pour mesurer une pression dans la chambre (2) ; et/ou
iv) est mise sous pression, de préférence à une pression comprise entre 0,1 et 20 bars, de préférence comprise entre 0,5 et 15 bars, de manière particulièrement préférée comprise entre 1 et 10 bars, notamment via une source d'air comprimé à laquelle la chambre (2) est reliée ; et/ou
v) contient un capteur d'humidité pour mesurer une humidité dans la chambre (2) ; et/ou
vi) contient un capteur d'humidité de matériau pour mesurer une humidité du matériau dans la chambre (2), de préférence un capteur d'humidité de matériau basé sur une mesure de résistance de courant ; et/ou
vii) est ajustée à une humidité déterminée, notamment via une source d'eau ou une source de vapeur à laquelle la chambre (2) est reliée.

9. Procédé selon l'une des revendications 7 ou 8, **caractérisé en ce que** l'unité de dosage (3)
i) est mobile dans une direction x, dans une direction y et dans une direction z, de préférence via une cinématique cartésienne, de manière particulièrement préférée en direction d'un sol (10) de la chambre (2) ; et/ou
ii) est connectée à une source de tension électrique pour fournir une tension électrique à l'unité de dosage (3) et/ou est connectée à une source d'ultrasons, la source de tension et/ou la source d'ultrasons étant de préférence utilisées pour libérer la au moins une enzyme (5) dans des cellules biologiques, de préférence par lyse des cellules biologiques ; et/ou
iii) est reliée de manière fluidique à un réservoir qui est adapté pour ajouter une substance dans l'unité de dosage (3), la substance étant de préférence appropriée pour effectuer une production et/ou libération de la ou des enzymes (5) dans des cellules biologiques, de préférence via un transport de la substance hors des cellules biologiques et/ou une lyse des cellules biologiques ; et/ou
iv) est équipée d'une tête de pulvérisation en direction de la chambre (2) ; et/ou
v) est équipée d'un capteur de température (12) pour mesurer une température dans l'unité de dosage (3) ; et/ou
vi) peut être tempérée ou est tempérée, de préférence peut être tempérée ou est tempérée à une température comprise entre 1 et 20°C, de préférence comprise entre 2 à 10°C, de manière particulièrement préférée comprise entre 3 et 5°C, l'unité de dosage (3) contenant notamment un système de refroidissement ; et/ou
vii) est équipée d'un capteur de pression pour mesurer une pression dans l'unité de dosage (3) ; et/ou
viii) peut être mise sous pression ou est mise sous pression, de préférence à une pression comprise entre 0,1 et 20 bars, de préférence comprise entre 0,5 et 15 bars, de manière particulièrement préférée comprise entre 1 et 10 bars, en particulier via de l'air comprimé provenant d'une source d'air comprimé à laquelle l'unité de dosage (3) est ou sera reliée ; et/ou
ix) est équipée d'un capteur d'humidité d'air pour mesurer une humidité d'air dans l'unité de dosage (3) ; et/ou
x) peut être réglée ou est réglée sur une humidité spécifique, l'unité de dosage (3) notamment via de l'eau et/ou de la vapeur provenant d'une source d'eau et/ou de vapeur à laquelle l'unité de dosage (3) est ou sera reliée ; et/ou
xi) est équipée d'un pH-mètre ; et/ou
xii) est reliée à un spectromètre UV-Vis et une mesure d'une absorption UV-Vis d'un milieu situé à l'intérieur de l'unité de dosage (3) est effectuée, de préférence une absorption à une longueur d'onde comprise entre 240 et 750 nm, de préférence comprise entre 280 nm et 700 nm ; et/ou
xiii) est équipée d'un capteur de niveau remplissage qui est approprié pour mesurer un volume d'une solution enzymatique dans l'unité de dosage (3) ; et/ou
xiv) est dotée de molécules qui favorisent les liaisons covalentes du matériau biologique, de préférence une molécule choisie dans le groupe constitué par des substrats enzymatiques, des cofacteurs enzymatiques, des molécules activées chimiquement et des mélanges de ceux-ci, les substrats enzymatiques étant choisis notamment parmi des glucides, des protéines, des peptides, des acides nucléiques, des lipides, des phénols, des phénylpropanoïdes et des mélanges de ceux-ci, facultativement des monomères, des oligomères et/ou des polymères de ces molécules, le cofacteur enzymatique est notamment la lysyl-tyrosyl-quinone et/ou les substrats enzymatiques sont notamment des molécules activées chimiquement ; et/ou
xv) ne contient aucune fibre naturelle, de préférence ne contient aucun matériau biologique (6) qui est lié de manière covalente par une réaction catalysée par ladite au moins une enzyme (5) ;
xvi) ne contient aucun minéral hygroscopique, de préférence ne contient aucun matériau hygroscopique ; et/ou
xvii) présente une fermeture pouvant ^ter ouverte en direction de la chambre (2), l'unité de commande (4) provoquant de préférence une ouverture de la fermeture pouvant être ouverte, de manière particulièrement préférée sur la base d'une commande provenant d'un utilisateur.

10. Procédé selon l'une des revendications 7 à 9, **caractérisé en ce que** la au moins une enzyme (5)
i) est sous forme dissoute ou solide, de préférence dissoute dans une solution tampon aqueuse ou un milieu de fermentation aqueux ou sous la forme d'un poudre solide ; et/ou
ii) est contenue par des filaments solides et/ou est immobilisée dans des filaments solides ; et/ou
iii) est contenue dans des cellules biologiques et/ou est contenue dans la membrane cellulaire de cellules biologiques, les cellules biologiques étant de préférence sélectionnées dans le groupe constitué par des cellules procaryotes, des cellules eucaryotes et des mélanges de celles-ci, de manière particulièrement préférée sélectionnées dans le groupe constitué par des cellules bactériennes, des cellules archées, des cellules fongiques, végétales ou animales et des mélanges de celles-ci ; et/ou
iv) est fournie dans un espace intérieur de l'unité de dosage (3) ; et/ou
v) est fournie dans la chambre (2) ; et/ou
vi) est fournie immobilisée sur une paroi externe de l'unité de dosage (3) faisant face à la chambre (2) ; et/ou
vii) est choisie dans le groupe constitué des laccases, des tyrosinases, des peroxydases, des phénoloxydases, des oxydases d'acides aminés, des transglutaminases et des mélanges de celles-ci.

11. Procédé selon l'une des revendications 7 à 10, **caractérisé en ce que** l'unité de commande (4)
i) régule une température de la chambre (2) et/ou de l'unité de dosage (3), de préférence à une température comprise entre 30 et 60°C, de manière particulièrement préférée comprise entre 30 et 50°C, pour la chambre (2) et/ ou à une Température comprise entre 1 et 20°C, de préférence comprise entre 2 et 10°C, de manière particulièrement préférée comprise entre 3 et 5°C pour l'unité de dosage (3), notamment via la commande d'un élément chauffant (7) et/ ou d'un système de refroidissement (8) de la chambre (2) et/ou de l'unité de dosage (3) ;
ii) va réguler une pression dans la chambre (2) et/ou dans l'unité de dosage (3), de préférence à une pression comprise entre 0,1 et 20 bars, de préférence comprise entre 0,5 et 15 bars, de manière particulièrement préférée comprise entre 1 à 10 bars, notamment via une commande d'une source d'air comprimé reliée à la chambre (2) et/ou à l'unité de dosage (3) ; et/ou
iii) va réguler une humidité dans la chambre (2) et/ou l'unité de dosage (3), notamment via une source d'eau ou une source de vapeur d'eau reliée à la chambre (2) et/ou à l'unité de dosage (3) ; et/ou
iv) va déterminer la valeur de pH d'un milieu dans lequel se trouve la au moins une enzyme (5), notamment via un pH-mètre de l'unité de dosage (3) ; et/ou
v) va déterminer une concentration de la au moins une enzyme (5) dans un milieu dans lequel se trouve la au moins une enzyme (5), notamment via un spectromètre UV qui est relié à l'unité de dosage (3) ; et/ou
vi) va déterminer un volume d'une solution enzymatique dans l'unité de dosage (3), notamment via un capteur de niveau de remplissage qui est contenu dans l'unité de dosage (3) ; et/ou
vii) va déterminer un temps d'exposition de la au moins une enzyme (5) à un matériau biologique (6) situé dans la chambre (2).

12. Procédé selon l'une des revendications 7 à 11, **caractérisé en ce que** l'imprimante 3D (1) est équipée d'une unité de rayonnement (9) qui est adaptée pour rayonner un rayonnement électromagnétique dans la chambre (2), l'unité de rayonnement (9) étant de préférence adaptée à cet effet,
a) pour rayonner une lumière infrarouge dans la chambre (2), l'unité de rayonnement (9) étant de manière particulièrement préférée choisie dans le groupe constitué d'une lampe IR, d'un laser IR et de combinaisons de ceux-ci ; et/ou
b) pour rayonner de la lumière UV dans la chambre (2), l'unité de rayonnement (9) étant choisie de manière particulièrement préférée dans le groupe constitué d'une lampe UV, d'un laser UV et de combinaisons de ceux-ci ;
dans lequel un certain temps après la mise en contact de la au moins une enzyme (5) avec le matériau biologique situé dans la chambre (2), pour rayonner sur le matériau biologique à l'aide de l'unité de rayonnement (9) au moins par endroits, de sorte que la au moins une enzyme (5) soit activée ou désactivée.

13. Extrudeuse, presse à injecter ou presse à mouler, contenant
a) une chambre pour recevoir un matériau, contenant facultativement un matériau biologique ;
b) une unité de dosage pour mettre en contact au moins une enzyme avec un matériau situé dans la chambre ; et
c) une unité de commande ;
**caractérisé en ce que** l'unité de dosage contient au moins une enzyme qui est adaptée pour catalyser une réaction qui est adaptée pour effectuer une liaison covalente d'un matériel biologique, et l'unité de commande est configurée pour mettre en contact ladite au moins une enzyme avec un matériau biologique situé dans la chambre de telle sorte que la quantité totale d'enzyme soit comprise entre 0,001 % en poids et < 0,01 % en poids, sur la base de la quantité totale de matériau biologique et d'enzyme dans la chambre.

14. Procédé d'extrusion, de moulage par compression ou de moulage par injection d'un matériau biologique, comprenant les étapes consistant à
a) fournir un matériau biologique dans une chambre d'une extrudeuse, d'une presse à mouler ou d'une presse à injecter, la chambre étant adaptée pour recevoir un matériau biologique ;
b) fournir une unité de dosage adaptée pour mettre en contact au moins une enzyme avec le matériau biologique situé dans la chambre ; et
c) fournir une unité de commande ;
**caractérisé en ce que** l'unité de dosage est dotée d'au moins une enzyme qui est adaptée pour catalyser une réaction qui est adapter pour effectuer une liaison covalente d'un matériau biologique, et l'unité de commande provoque une mise en contact de la au moins une enzyme avec le matériau biologique situé dans la chambre de telle sorte que la quantité totale d'enzyme soit comprise entre 0,001 % en poids et < 0,01 % en poids, sur la base de la quantité totale de matériau biologique et d'enzyme dans la chambre.

15. Matériau biologique produit par un procédé selon l'une des revendications 7 à 12, dans lequel plusieurs couches de matériau biologique sont reliées entre elles par action enzymatique,
**caractérisé en ce que** le matériau biologique présente une structure en couches, l'enzyme étant située entre les couches du matériau biologique relié, qui est adaptée pour catalyser une réaction qui est adaptée pour provoquer une liaison covalente du matériau biologique,
dans lequel la quantité totale de l'enzyme qui est adaptée pour catalyser une réaction capable d'effectuer une liaison covalente de matière biologique est comprise entre 0,001 % en poids et < 0,01 % en poids sur la quantité totale d'enzyme et de matériau biologique.
